# EUROPEAN PATENT APPLICATION

(11) **EP 4 357 358 A1**
(43) Date of publication of application: **24.04.2024**
(21) Application number: 22824309.3
(22) Date of filing: 17.06.2022
(51) Int. Cl.: C07K 14/605, A61K 38/26, A61P 3/10

(54) **GLUCAGON ANALOG AND MEDICAL USE THEREOF**

(30) Priority: 18.06.2021 CN 202110676681
(71) Applicant: Beijing Tuo Jie Biopharmaceutical Co. Ltd., Beijing 102206 (CN)
(72) Inventor: LIU, Weibing, Beijing 102206 (CN); HUANG, Xuchao, Beijing 102206 (CN); ZHANG, Xiaoqian, Beijing 102206 (CN); WU, Fangzhou, Beijing 102206 (CN); WANG, Lei, Beijing 102206 (CN); QU, Liang, Beijing 102206 (CN)
(74) Representative: Dragotti & Associati S.R.L.
(86) International application number: PCT/CN2022/099357
(87) International publication number: WO 2022/262837

(57) **Abstract**

A glucagon analog and the medical use thereof. Specifically, the glucagon analog has a significantly improved in vitro activity, excellent physical/chemical stability, and high solubility, and can be used to treat metabolic diseases such as hypoglycemia, obesity, and diabetes.

## Description

The present application claims priority to Chinese Patent Application No. 202110676681.3 filed on June 18, 2021.

### TECHNICAL FIELD

The present disclosure relates to glucagon analogs and medical use thereof. The glucagon analogs disclosed herein can be used for treating or alleviating metabolic diseases such as hypoglycemia, obesity, diabetes, and the like.

### BACKGROUND

Controlling the balance of blood glucose in the body is extremely important to the metabolism. In a normal state, the blood glucose level in humans is maintained in homeostasis by the regulation of a number of different polypeptide hormones, mainly insulin and glucagon. Blood glucose imbalance may lead to complications. When the blood glucose concentration is lower than the normal level, hypoglycemia occurs. Cells in the islets produce glucagon, which up-regulates the blood glucose level to the normal range. Mild or moderate hypoglycemia often causes autonomic or neurogenic hypoglycemia symptoms that can be recognized by the patient, and thus does not require the help of others. When the blood glucose concentration is reduced by other drugs, diseases, or defects in hormone or enzyme (such as treatment of a series of clinical severe hypoglycemic symptoms caused by diabetes, renal failure, specific tumors, liver diseases, hypothyroidism, hereditary metabolic defects, septicemia, and reactive hypoglycemia), glucagon may not be adequate to restore a normal blood glucose level, which may even be life-threatening. Such situations usually cause impaired cognitive or physical functionality and thus limited self-treatment capability, and therefore, may need the help of others.

At present, in the prevention and treatment of hypoglycemia, the preferred therapy for completely conscious hypoglycemia patients is oral saccharide food; those of impaired consciousness may need intravenous administration of 50% glucose solution or intramuscular administration of 0.5 mg to 2 mg of glucagon, with the aid of others. However, the intravenous injection of 50% glucose solution may be difficult for a person without expertise. Overdose can cause rebound hyperglycemia, and leakage of the glucose injection may lead to local tissue damage. Thus, in a non-hospital setting, the best treatment strategy for a patient with severe hypoglycemia and disturbance in consciousness is subcutaneous or intramuscular injection of glucagon. Glucagon has also been used clinically as a first-aid drug for severe hypoglycemia. Based on the effect of glucagon in lowering lipid levels and promoting energy expenditure, its dual receptor agonist with incretin may further benefit diabetic patients with obesity.

Glucagon is a linear polypeptide of 29 amino acids secreted by pancreatic alpha-cells. It controls the production of glucose and ketone bodies in the liver. Glucagon is secreted in the night and between meals to increase blood glucose concentration by increasing glucose production from amino acid precursors (gluconeogenesis) and promoting glycogen degradation to glucose (glycolysis) which results in increased glucose efflux in the liver. Glucagon, together with insulin secreted by the pancreatic beta cells precisely controls the blood glucose balance in the body. Glucagon, in addition to the therapeutic effect on hypoglycemia, can also suppress appetite and activate hormone-sensitive lipase of adipocytes to promote lipolysis, thereby exhibiting an anti-obesity effect.

Glucagon is an important component of the defense mechanism against hypoglycemia, and low doses of glucagon can prevent insulin-induced hypoglycemia and improve the ability to recover from hypoglycemia. A low dose of glucagon can also induce satiety to suppress appetite while activating hormone-sensitive lipase of adipocytes and promoting lipolysis, and thereby has a potential anti-obesity effect and potential for treating overweight or obesity of many patients with type II diabetes mellitus.

However, native glucagon has a low solubility in aqueous solutions at neutral pH. In addition, glucagon may comprise amino acids or amino acid sequences that are prone to deamidation, oxidation, formation of cyclic imine intermediates in amino acid residue side chains, and amino acids or amino acid sequences that may undergo isomerization, peptide chain cleavage, and the like. Therefore, glucagon may become instable over time, and form gel or fibril within several hours to several days, showing extremely poor chemical and physical stabilities. Accordingly, there is a need to develop a novel glucagon analog having excellent solubility and good physical/chemical stability to meet the requirements of the development of injections.

Glucagon injections of the first generation, such as emergent glucagon kit (Glucagon^{®}, Eli Lilly) and glucagon kit (Glucagon^{®}, Novo Nordisk A/S), are limited by the unsatisfying druggability of glucagon, and are usually preserved in the form of lyophilized powder, which is reconstituted in a solvent before use in subcutaneous/intramuscular administration and thus inconvenient for patients and carers without expertise.

Glucagon injections of the second generation, such as nasal human glucagon powder (BAQSIMI^{®}, Eli Lilly) and pre-filled human glucagon syringe (GVOKE HYPOPEN^{®}, XERIS), are improved preparations, and feature significantly improved convenience for usage by patients and carers without expertise while retaining the efficacy. However, BAQSIMI^{®} is administrated by nasal spray, possessing a delayed effect, a dose three times that of an intramuscular injection, and adverse effects. GVOKE HYPOPEN^{®} is formulated in DMSO, and may cause significant injection site discomfort and other adverse effects despite the capability of subcutaneous administration. Dasiglucagon (Zealand Pharma) comprises 7 modified amino acids on the basis of the native human glucagon, and thus has improved solubility and physical/chemical stability without affecting the activity, and the capability of subcutaneous administration in the form of an injection. Dasiglucagon has recently been approved by the U.S. Food and Drug Administration.

The related art is still in urgent need for glucagon analogs having high activity, excellent solubility, and good physical/chemical stability. The present disclosure provides a group of glucagon analogs with high activity (compared with the native glucagon), high solubility in aqueous solutions (particularly at physiological pH), and improved stability (including physical and chemical stability). Compared with glucagon analogs in the prior art, the glucagon analog disclosed herein has stronger in-vitro glucagon receptor agonist activity, and is greatly helpful in reducing the dosage and frequency of subcutaneous administration, improving the convenience for hypoglycemia patients, and reducing the financial burden of the patients. The glucagon analog disclosed herein can be administered alone or in combination with other therapeutic agents in methods for treating metabolic diseases or conditions such as hypoglycemia, obesity, diabetes, and the like.

### SUMMARY

The present disclosure provides a glucagon analog, or a pharmaceutically acceptable salt and/or solvate thereof, a pharmaceutical composition comprising same, a polynucleotide encoding the glucagon analog, a vector comprising the polynucleotide, and a host cell comprising the polynucleotide or the vector. Also provided are a method for treating a disease or alleviating a condition (such as hypoglycemia, obesity, diabetes, and other metabolic diseases or conditions) by using the glucagon analog or the pharmaceutically acceptable salt and/or solvate thereof or the pharmaceutical compositions comprising same, and related pharmaceutical use.

### Glucagon Analog, or Pharmaceutically Acceptable Salt and/or Solvate Thereof

The present disclosure provides a glucagon analog having a structure of formula (I), or a pharmaceutically acceptable salt and/or solvate thereof:

R1-His-Ser-X3-Gly-Thr-Phe-Thr-Ser-Asp-Tyr-Ser-Lys-Tyr-Leu-X15-X16-X17-Arg-Ala-
X20-X21-Phe-Val-X24-Trp-Leu-X27-X28-Thr-R2 (SEQ ID NO: 50)
(I)

The present disclosure further provides a glucagon analog comprising a structure of formula (I), or a pharmaceutically acceptable salt and/or solvate thereof, wherein R₁ and/or R₂ is absent;
wherein:
R₁ is hydrogen, C₁₋₄ alkyl, acetyl, formyl, benzoyl, trifluoroacetyl, or pGlu;
R₂ is -OH or -NH₂;
X₃, X₁₅, X₁₆, X₂₀, X₂₁, X₂₄, X₂₇, and X₂₈ are independently selected from the group consisting of any natural and unnatural amino acid residues; X₁₇ is Aib.

In some embodiments, X₃ is selected from the group consisting of His, Dap (Ac), and Gln; X₁₅ is selected from the group consisting of Asp and Glu; X₁₆ is selected from the group consisting of Ser, Thr, Leu, Val, Ile, and alpha-methyl-Ser; X₁₇ is Aib; X₂₀ is selected from the group consisting of Ala, Gln, Glu, Ser, Thr, and Lys; X₂₁ is selected from the group consisting of Asp and Glu; X₂₄ is selected from the group consisting of Ala, Gin, Ser, Glu, alpha-methyl-Ser, and Arg; X₂₇ is selected from the group consisting of Met, Glu, Nle, Leu, and Ser; X₂₈ is selected from the group consisting of Asn, Glu, and Ser.

In some embodiments, X₃ is selected from the group consisting of His, Dap (Ac), and Gln; X₁₅ is Asp; X₁₆ is selected from the group consisting of Thr, Leu, Val, and Ile; X₁₇ is Aib; X₂₀ is selected from the group consisting of Ala, Gln, Glu, Ser, Thr, and Lys; X₂₁ is selected from the group consisting of Asp and Glu; X₂₄ is selected from the group consisting of Ala, Gln, Ser, Glu, alpha-methyl-Ser, and Arg; X₂₇ is selected from the group consisting of Met, Glu, Nle, Leu, and Ser; X₂₈ is selected from the group consisting of Asn, Glu, and Ser.

In some embodiments, X₃ is selected from the group consisting of His, Dap (Ac), and Gln; X₁₅ is selected from the group consisting of Asp and Glu; X₁₆ is selected from the group consisting of Ser, Thr, Leu, Val, Ile, and alpha-methyl-Ser; X₁₇ is Aib; X₂₀ is Gin; X₂₁ is selected from the group consisting of Asp and Glu; X₂₄ is selected from the group consisting of Ala, Gin, Ser, Glu, alpha-methyl-Ser, and Arg; X₂₇ is selected from the group consisting of Met, Glu, Nle, Leu, and Ser; X₂₈ is selected from the group consisting of Asn, Glu, and Ser.

In some embodiments, X₃ is selected from the group consisting of His, Dap (Ac), and Gln; X₁₅ is selected from the group consisting of Asp and Glu; X₁₆ is selected from the group consisting of Ser, Thr, Leu, Val, Ile, and alpha-methyl-Ser; X₁₇ is Aib; X₂₀ is selected from the group consisting of Ala, Gin, Glu, Ser, Thr, and Lys; X₂₁ is selected from the group consisting of Asp and Glu; X₂₄ is selected from the group consisting of Gln and Glu; X₂₇ is selected from the group consisting of Met, Glu, Nle, Leu, and Ser; X₂₈ is selected from the group consisting of Asn, Glu, and Ser.

In some embodiments, X₃ is selected from the group consisting of His, Dap (Ac), and Gln; X₁₅ is selected from the group consisting of Asp and Glu; X₁₆ is selected from the group consisting of Ser, Thr, Leu, Val, Ile, and alpha-methyl-Ser; X₁₇ is Aib; X₂₀ is selected from the group consisting of Ala, Gin, Glu, Ser, Thr, and Lys; X₂₁ is selected from the group consisting of Asp and Glu; X₂₄ is selected from the group consisting of Ala, Gin, Ser, Glu, alpha-methyl-Ser, and Arg; X₂₇ is Glu; X₂₈ is Ser.

In some embodiments, X₃ is selected from the group consisting of His, Dap (Ac), and Gln; X₁₅ is Asp; X₁₆ is selected from the group consisting of Thr, Leu, Val, and Ile; X₁₇ is Aib; X₂₀ is Gin; X₂₁ is selected from the group consisting of Asp and Glu; X₂₄ is selected from the group consisting of Ala, Gin, Ser, Glu, alpha-methyl-Ser, and Arg; X₂₇ is selected from the group consisting of Met, Glu, Nle, Leu, and Ser; X₂₈ is selected from the group consisting ofAsn, Glu, and Ser.

In some embodiments, X₃ is selected from the group consisting of His, Dap (Ac), and Gln; X₁₅ is Asp; X₁₆ is selected from the group consisting of Thr, Leu, Val, and Ile; X₁₇ is Aib; X₂₀ is selected from the group consisting of Ala, Gln, Glu, Ser, Thr, and Lys; X₂₁ is selected from the group consisting of Asp and Glu; X₂₄ is selected from the group consisting of Gin and Glu; X₂₇ is selected from the group consisting of Met, Glu, Nle, Leu, and Ser; X₂₈ is selected from the group consisting of Asn, Glu, and Ser.

In some embodiments, X₃ is selected from the group consisting of His, Dap (Ac), and Gln; X₁₅ is Asp; X₁₆ is selected from the group consisting of Thr, Leu, Val, and Ile; X₁₇ is Aib; X₂₀ is selected from the group consisting of Ala, Gin, Glu, Ser, Thr, and Lys; X₂₁ is selected from the group consisting of Asp and Glu; X₂₄ is selected from the group consisting of Ala, Gln, Ser, Glu, alpha-methyl-Ser, and Arg; X₂₇ is Glu; X₂₈ is Ser.

In some embodiments, X₃ is selected from the group consisting of His, Dap (Ac), and Gln; X₁₅ is selected from the group consisting of Asp and Glu; X₁₆ is selected from the group consisting of Ser, Thr, Leu, Val, Ile, and alpha-methyl-Ser; X₁₇ is Aib; X₂₀ is Gln; X₂₁ is selected from the group consisting of Asp and Glu; X₂₄ is selected from the group consisting of Gln and Glu; X₂₇ is selected from the group consisting of Met, Glu, Nle, Leu, and Ser; X₂₈ is selected from the group consisting of Asn, Glu, and Ser.

In some embodiments, X₃ is selected from the group consisting of His, Dap (Ac), and Gln; X₁₅ is selected from the group consisting of Asp and Glu; X₁₆ is selected from the group consisting of Ser, Thr, Leu, Val, Ile, and alpha-methyl-Ser; X₁₇ is Aib; X₂₀ is Gln; X₂₁ is selected from the group consisting of Asp and Glu; X₂₄ is selected from the group consisting of Ala, Gin, Ser, Glu, alpha-methyl-Ser, and Arg; X₂₇ is Glu; X₂₈ is Ser.

In some embodiments, X₃ is selected from the group consisting of His, Dap (Ac), and Gin; X₁₅ is selected from the group consisting of Asp and Glu; X₁₆ is selected from the group consisting of Ser, Thr, Leu, Val, Ile, and alpha-methyl-Ser; X₁₇ is Aib; X₂₀ is selected from the group consisting of Ala, Gln, Glu, Ser, Thr, and Lys; X₂₁ is selected from the group consisting of Asp and Glu; X₂₄ is selected from the group consisting of Gln and Glu; X₂₇ is Glu; X₂₈ is Ser.

In some embodiments, X₃ is selected from the group consisting of His, Dap (Ac), and Gln; X₁₅ is Asp; X₁₆ is selected from the group consisting of Thr, Leu, Val, and Ile; X₁₇ is Aib; X₂₀ is Gln; X₂₁ is selected from the group consisting of Asp and Glu; X₂₄ is selected from the group consisting of Gin and Glu; X₂₇ is Glu; X₂₈ is Ser.

In some embodiments, X₃ is selected from the group consisting of His and Gln; X₁₅ is Asp; X₁₆ is selected from the group consisting of Thr, Leu, Val, and Ile; X₁₇ is Aib; X₂₀ is Gln; X₂₁ is Glu; X₂₄ is selected from the group consisting of Gln and Glu; X₂₇ is Glu; X₂₈ is Ser.

In some embodiments, R₁ is hydrogen; R₂ is -OH or -NH₂. In some embodiments, R₁ is hydrogen; R₂ is -OH.

The pharmaceutically acceptable salt and/or solvate disclosed herein are/is selected from the group consisting of: an inorganic salt and an organic salt.

The glucagon analog disclosed herein may form a corresponding salt by a reaction with an acidic compound or a basic compound.

The "corresponding salt formed by a reaction with an acidic compound" refers to salts that are capable of retaining the biological effectiveness of free bases without having any undesirable effects and that are formed with inorganic acids or organic acids. The inorganic acid salts include, but are not limited to, hydrochlorides, hydrobromides, sulfates, nitrates, phosphates, etc.; the organic acid salts include, but are not limited to, formates, acetates, 2,2-dichloroacetates, trifluoroacetates, propionates, caproates, caprylates, caprates, undecenates, glycolates, gluconates, lactates, sebacates, adipates, glutarates, malonates, oxalates, maleates, succinates, fumarates, tartrates, citrates, palmitates, stearates, oleates, cinnamates, laurates, malates, glutamates, pyroglutamates, aspartates, benzoates, methanesulfonates, benzenesulfonates, p-toluenesulfonates, alginates, ascorbates, salicylates, 4-aminosalicylates, napadisylates, etc. Such salts can be prepared using methods known in the art.

The "corresponding salt formed by a reaction with a basic compound" refers to salts that are capable of retaining the biological effectiveness of free acids without having any undesirable effects and that are formed with inorganic bases or organic bases. The salts derived from inorganic bases include, but are not limited to: sodium salts, potassium salts, lithium salts, ammonium salts, calcium salts, magnesium salts, iron salts, zinc salts, copper salts, manganese salts, aluminum salts, etc. The salts derived from organic bases include, but are not limited to, salts of the following: primary, secondary and tertiary amines, substituted amines, and cyclic amines; for example, ammonia, isopropylamine, trimethylamine, diethylamine, triethylamine, tripropylamine, ethanolamine, ethylenediamine, diethanolamine, triethanolamine, dimethylethanolamine, 2-dimethylaminoethanol, 2-diethylaminoethanol, dicyclohexylamine, lysine, arginine, histidine, caffeine, procaine, choline, betaine, glucosamine, methylglucamine, theobromine, purine, piperazine, piperidine, N-ethylpiperidine, polyamine resins, etc. Preferred organic bases include isopropylamine, diethylamine, ethanolamine, trimethylamine, dicyclohexylamine, choline, and caffeine. Such salts can be prepared using methods known in the art.

The term "solvate" refers to a complex of the glucagon analog or the pharmaceutically acceptable salt thereof disclosed herein and a suitable solvent. Non-limiting examples of the solvent include: water, ethanol, acetonitrile, isopropanol, DMSO, and ethyl acetate. In a specific embodiment, the solvate is a hydrate.

In some embodiments, the present disclosure provides a glucagon analog, or a pharmaceutically acceptable salt and/or solvate thereof, wherein the glucagon analog is selected from the group consisting of any of the compounds set forth in SEQ ID NOs: 1-49.

In some specific embodiments, the present disclosure provides a glucagon analog, or a pharmaceutically acceptable salt and/or solvate thereof, wherein the glucagon analog is selected from the group consisting of the following compounds:
H-HSQGTFTSDYSKYLDTAibRAQEFVQWLEST-OH (SEQ ID NO: 39);
H-HSQGTFTSDYSKYLDLAibRAQEFVQWLEST-OH (SEQ ID NO: 40);
H-HSQGTFTSDYSKYLDVAibRAQEFVQWLEST-OH (SEQ ID NO: 41);
H-HSQGTFTSDYSKYLDIAibRAQEFVQWLEST-OH (SEQ ID NO: 42);
H-HSHGTFTSDYSKYLDLAibRAQEFVQWLEST-OH (SEQ ID NO: 43); and
H-HSHGTFTSDYSKYLDLAibRAQEFVEWLEST-OH (SEQ ID NO: 47).

In the sequence of each of the glucagon analogs disclosed herein, the C-terminal "-OH" moiety may be substituted by the C-terminal "-NH₂".

In some specific embodiments, the present disclosure provides a glucagon analog, or a pharmaceutically acceptable salt and/or solvate thereof, wherein the glucagon analog is selected from the group consisting of any of the structures set forth in SEQ ID NOs: 39. 40, 41, 42, 43, and 47, wherein the N-terminal H and/or C-terminal OH in SEQ ID NOs: 39. 40, 41, 42, 43, and 47 are absent.

The present disclosure provides a glucagon analog having a structure of formula (I), or a pharmaceutically acceptable salt and/or solvate thereof:

R1-His-Ser-X3-Gly-Thr-Phe-Thr-Ser-Asp-Tyr-Ser-Lys-Tyr-Leu-X15-X16-X17-Arg-Ala-
X20-X21-Phe-Val-X24-Trp-Leu-X27-X28-Thr-R2 (SEQ ID NO: 50)
(I)

The present disclosure further provides a glucagon analog comprising a structure of formula (I), or a pharmaceutically acceptable salt and/or solvate thereof, wherein R₁ and/or R₂ are/is absent;
wherein:
R₁ is hydrogen, C₁₋₄ alkyl, acetyl, formyl, benzoyl, trifluoroacetyl, or pGlu;
R₂ is -OH or -NH₂;
X₃, X₁₇, X₂₀, X₂₁, X₂₄, X₂₇, and X₂₈ are independently selected from the group consisting of any natural and unnatural amino acid residues; X₁₅ is Asp; X₁₆ is selected from the group consisting of Ser, Thr, Leu, Val, Ile, and alpha-methyl-Ser, for example, selected from the group consisting of Thr, Leu, Val, and Ile.

In some embodiments, X₃ is selected from the group consisting of His, Dap (Ac), and Gln; X₁₅ is Asp; X₁₆ is selected from the group consisting of Ser, Thr, Leu, Val, Ile, and alpha-methyl-Ser; X₁₇ is selected from the group consisting of Aib and Ala; X₂₀ is not Glu; X₂₁ is selected from the group consisting of Asp and Glu; X₂₄ is selected from the group consisting of Ala, Gln, Ser, Glu, alpha-methyl-Ser, and Arg; X₂₇ is selected from the group consisting of Met, Glu, Nle, Leu, and Ser; X₂₈ is selected from the group consisting of Asn, Glu, and Ser.

In some embodiments, X₃ is selected from the group consisting of His, Dap (Ac), and Gin; X₁₅ is Asp; X₁₆ is selected from the group consisting of Thr, Leu, Val, and Ile; X₁₇ is selected from the group consisting of Aib and Ala; X₂₀ is not Glu; X₂₁ is selected from the group consisting of Asp and Glu; X₂₄ is selected from the group consisting of Ala, Gln, Ser, Glu, alpha-methyl-Ser, and Arg; X₂₇ is selected from the group consisting of Met, Glu, Nle, Leu, and Ser; X₂₈ is selected from the group consisting of Asn, Glu, and Ser.

In some embodiments, X₃ is selected from the group consisting of His, Dap (Ac), and Gln; X₁₅ is Asp; X₁₆ is selected from the group consisting of Thr, Leu, Val, and Ile; X₁₇ is Aib; X₂₀ is selected from the group consisting of Ala, Gin, Glu, Ser, Thr, and Lys; X₂₁ is selected from the group consisting of Asp and Glu; X₂₄ is selected from the group consisting of Ala, Gln, Ser, Glu, alpha-methyl-Ser, and Arg; X₂₇ is selected from the group consisting of Met, Glu, Nle, Leu, and Ser; X₂₈ is selected from the group consisting of Asn, Glu, and Ser.

In some embodiments, X₃ is selected from the group consisting of His, Dap (Ac), and Gln; X₁₅ is Asp; X₁₆ is selected from the group consisting of Thr, Leu, Val, and Ile; X₁₇ is Aib; X₂₀ is Gin; X₂₁ is selected from the group consisting of Asp and Glu; X₂₄ is selected from the group consisting of Ala, Gln, Ser, Glu, alpha-methyl-Ser, and Arg; X₂₇ is selected from the group consisting of Met, Glu, Nle, Leu, and Ser; X₂₈ is selected from the group consisting of Asn, Glu, and Ser.

In some embodiments, X₃ is selected from the group consisting of His, Dap (Ac), and Gln; X₁₅ is Asp; X₁₆ is selected from the group consisting of Thr, Leu, Val, and Ile; X₁₇ is Aib; X₂₀ is not Glu; X₂₁ is selected from the group consisting of Asp and Glu; X₂₄ is selected from the group consisting of Gln and Glu; X₂₇ is selected from the group consisting of Met, Glu, Nle, Leu, and Ser; X₂₈ is selected from the group consisting of Asn, Glu, and Ser.

In some embodiments, X₃ is selected from the group consisting of His, Dap (Ac), and Gln; X₁₅ is Asp; X₁₆ is selected from the group consisting of Thr, Leu, Val, and Ile; X₁₇ is Aib; X₂₀ is not Glu; X₂₁ is selected from the group consisting of Asp and Glu; X₂₄ is selected from the group consisting of Ala, Gln, Ser, Glu, alpha-methyl-Ser, and Arg; X₂₇ is Glu; X₂₈ is Ser.

In some embodiments, X₃ is selected from the group consisting of His, Dap (Ac), and Gln; X₁₅ is Asp; X₁₆ is selected from the group consisting of Thr, Leu, Val, and Ile; X₁₇ is Aib; X₂₀ is not Glu; X₂₁ is selected from the group consisting of Asp and Glu; X₂₄ is selected from the group consisting of Gln and Glu; X₂₇ is Glu; X₂₈ is Ser.

In some embodiments, X₃ is selected from the group consisting of His, Dap (Ac), and Gln; X₁₅ is Asp; X₁₆ is selected from the group consisting of Thr, Leu, Val, and Ile; X₁₇ is Aib; X₂₀ is selected from the group consisting of Ala, Gin, Glu, Ser, Thr, and Lys; X₂₁ is selected from the group consisting of Asp and Glu; X₂₄ is selected from the group consisting of Gln and Glu; X₂₇ is Glu; X₂₈ is Ser.

In some embodiments, X₃ is selected from the group consisting of His, Dap (Ac), and Gln; X₁₅ is Asp; X₁₆ is selected from the group consisting of Thr, Leu, Val, and Ile; X₁₇ is Aib; X₂₀ is Gin; X₂₁ is selected from the group consisting of Asp and Glu; X₂₄ is selected from the group consisting of Gin and Glu; X₂₇ is Glu; X₂₈ is Ser.

In some embodiments, X₃ is selected from the group consisting of His and Gln; X₁₅ is Asp; X₁₆ is selected from the group consisting of Thr, Leu, Val, and Ile; X₁₇ is Aib; X₂₀ is Gln; X₂₁ is Glu; X₂₄ is selected from the group consisting of Gln and Glu; X₂₇ is Glu; X₂₈ is Ser.

In some embodiments, R₁ is hydrogen; R₂ is -OH or -NH₂. In some embodiments, R₁ is hydrogen; R₂ is -OH.

The present disclosure provides a glucagon analog, or a pharmaceutically acceptable salt and/or solvate thereof, wherein the glucagon analog comprises a structure of formula (I):

R1-His-Ser-X3-Gly-Thr-Phe-Thr-Ser-Asp-Tyr-Ser-Lys-Tyr-Leu-X15-X16-X17-Arg-Ala-
X20-X21-Phe-Val-X24-Trp-Leu-X27-X28-Thr-R2 (SEQ ID NO: 50)
(I)

The present disclosure further provides a glucagon analog comprising a structure of formula (I), or a pharmaceutically acceptable salt and/or solvate thereof, wherein R₁ and/or R₂ are/is absent;
wherein:
R₁ is hydrogen, C₁₋₄ alkyl, acetyl, formyl, benzoyl, trifluoroacetyl, or pGlu;
R₂ is -OH or -NH₂;
X₃, X₁₅, X₁₆, X₁₇, X₂₁, X₂₄, X₂₇, and X₂₈ are independently selected from the group consisting of any natural and unnatural amino acid residues; X₂₀ is Gln.

In some embodiments, X₃ is selected from the group consisting of His, Dap (Ac), and Gln; X₁₅ is selected from the group consisting of Asp and Glu; X₁₆ is selected from the group consisting of Ser, Thr, Leu, Val, Ile, and alpha-methyl-Ser; X₁₇ is selected from the group consisting of Aib and Ala; X₂₀ is Gln; X₂₁ is selected from the group consisting of Asp and Glu; X₂₄ is selected from the group consisting of Ala, Gin, Ser, Glu, alpha-methyl-Ser, and Arg; X₂₇ is selected from the group consisting of Met, Glu, Nle, Leu, and Ser; X₂₈ is selected from the group consisting of Asn, Glu, and Ser.

In some embodiments, X₃ is selected from the group consisting of His, Dap (Ac), and Gln; X₁₅ is selected from the group consisting of Asp and Glu; X₁₆ is selected from the group consisting of Ser, Thr, Leu, Val, Ile, and alpha-methyl-Ser; X₁₇ is selected from the group consisting of Aib and Ala; X₂₀ is Gin; X₂₁ is selected from the group consisting of Asp and Glu; X₂₄ is selected from the group consisting of Gln and Glu; X₂₇ is selected from the group consisting of Met, Glu, Nle, Leu, and Ser; X₂₈ is selected from the group consisting of Asn, Glu, and Ser.

In some embodiments, X₃ is selected from the group consisting of His, Dap (Ac), and Gln; X₁₅ is Asp; X₁₆ is selected from the group consisting of Thr, Leu, Val, and Ile; X₁₇ is selected from the group consisting of Aib and Ala; X₂₀ is Gin; X₂₁ is selected from the group consisting of Asp and Glu; X₂₄ is selected from the group consisting of Ala, Gln, Ser, Glu, alpha-methyl-Ser, and Arg; X₂₇ is selected from the group consisting of Met, Glu, Nle, Leu, and Ser; X₂₈ is selected from the group consisting of Asn, Glu, and Ser.

In some embodiments, X₃ is selected from the group consisting of His, Dap (Ac), and Gln; X₁₅ is selected from the group consisting of Asp and Glu; X₁₆ is selected from the group consisting of Ser, Thr, Leu, Val, Ile, and alpha-methyl-Ser; X₁₇ is selected from the group consisting of Aib and Ala; X₂₀ is Gin; X₂₁ is selected from the group consisting of Asp and Glu; X₂₄ is selected from the group consisting of Ala, Gln, Ser, Glu, alpha-methyl-Ser, and Arg; X₂₇ is Glu; X₂₈ is Ser.

In some embodiments, X₃ is selected from the group consisting of His, Dap (Ac), and Gln; X₁₅ is Asp; X₁₆ is selected from the group consisting of Thr, Leu, Val, and Ile; X₁₇ is selected from the group consisting of Aib and Ala; X₂₀ is Gln; X₂₁ is selected from the group consisting of Asp and Glu; X₂₄ is selected from the group consisting of Gln and Glu; X₂₇ is selected from the group consisting of Met, Glu, Nle, Leu, and Ser; X₂₈ is selected from the group consisting of Asn, Glu, and Ser.

In some embodiments, X₃ is selected from the group consisting of His, Dap (Ac), and Gln; X₁₅ is Asp; X₁₆ is selected from the group consisting of Thr, Leu, Val, and Ile; X₁₇ is selected from the group consisting of Aib and Ala; X₂₀ is Gln; X₂₁ is selected from the group consisting of Asp and Glu; X₂₄ is selected from the group consisting of Ala, Gln, Ser, Glu, alpha-methyl-Ser, and Arg; X₂₇ is Glu; X₂₈ is Ser.

In some embodiments, X₃ is selected from the group consisting of His, Dap (Ac), and Gln; X₁₅ is selected from the group consisting of Asp and Glu; X₁₆ is selected from the group consisting of Ser, Thr, Leu, Val, Ile, and alpha-methyl-Ser; X₁₇ is selected from the group consisting of Aib and Ala; X₂₀ is Gln; X₂₁ is selected from the group consisting of Asp and Glu; X₂₄ is selected from the group consisting of Gln and Glu; X₂₇ is Glu; X₂₈ is Ser.

In some embodiments, X₃ is selected from the group consisting of His, Dap (Ac), and Gln; X₁₅ is Asp; X₁₆ is selected from the group consisting of Thr, Leu, Val, and Ile; X₁₇ is selected from the group consisting of Aib and Ala; X₂₀ is Gln; X₂₁ is selected from the group consisting of Asp and Glu; X₂₄ is selected from the group consisting of Gln and Glu; X₂₇ is Glu; X₂₈ is Ser.

In some embodiments, X₃ is selected from the group consisting of His and Gln; X₁₅ is Asp; X₁₆ is selected from the group consisting of Thr, Leu, Val, and Ile; X₁₇ is Aib; X₂₀ is Gin; X₂₁ is Glu; X₂₄ is selected from the group consisting of Gln and Glu; X₂₇ is Glu; X₂₈ is Ser.

In some embodiments, R₁ is hydrogen; R₂ is -OH or -NH₂. In some embodiments, R₁ is hydrogen; R₂ is -OH.

The present disclosure provides a glucagon analog having a structure of formula (I), or a pharmaceutically acceptable salt and/or solvate thereof:

R1-His-Ser-X3-Gly-Thr-Phe-Thr-Ser-Asp-Tyr-Ser-Lys-Tyr-Leu-X15-X16-X17-Arg-Ala-
X20-X21-Phe-Val-X24-Trp-Leu-X27-X28-Thr-R2 (SEQ ID NO: 50)
(I)

The present disclosure further provides a glucagon analog comprising a structure of formula (I), or a pharmaceutically acceptable salt and/or solvate thereof, wherein R₁ and/or R₂ are/is absent;
wherein:
R₁ is hydrogen, C₁₋₄ alkyl, acetyl, formyl, benzoyl, trifluoroacetyl, or pGlu;
R₂ is -OH or -NH₂;
X₃, X₁₅, X₁₆, X₁₇, X₂₁, X₂₇, and X₂₈ are independently selected from the group consisting of any natural and unnatural amino acid residues; X₂₀ is Glu, and X₂₄ is Arg. In some embodiments, X₃ is selected from the group consisting of His, Dap (Ac), and Gln; X₁₅ is selected from the group consisting of Asp and Glu; X₁₆ is selected from the group consisting of Ser, Thr, Leu, Val, Ile, and alpha-methyl-Ser; X₁₇ is Aib; X₂₀ is Glu; X₂₁ is selected from the group consisting of Asp and Glu; X₂₄ is Arg; X₂₇ is selected from the group consisting of Met, Glu, Nle, Leu, and Ser; X₂₈ is selected from the group consisting of Asn, Glu, and Ser.

In some embodiments, X₃ is selected from the group consisting of His, Dap (Ac), and Gin; X₁₅ is Asp; X₁₆ is selected from the group consisting of Thr, Leu, Val, and Ile; X₁₇ is Aib; X₂₀ is Glu; X₂₁ is selected from the group consisting of Asp and Glu; X₂₄ is Arg; X₂₇ is selected from the group consisting of Met, Glu, Nle, Leu, and Ser; X₂₈ is selected from the group consisting of Asn, Glu, and Ser.

In some embodiments, X₃ is selected from the group consisting of His, Dap (Ac), and Gln; X₁₅ is selected from the group consisting of Asp and Glu; X₁₆ is selected from the group consisting of Ser, Thr, Leu, Val, Ile, and alpha-methyl-Ser; X₁₇ is Aib; X₂₀ is Glu; X₂₁ is selected from the group consisting of Asp and Glu; X₂₄ is Arg; X₂₇ is Glu; X₂₈ is Ser.

In some embodiments, X₃ is selected from the group consisting of His, Dap (Ac), and Gln; X₁₅ is Asp; X₁₆ is selected from the group consisting of Thr, Leu, Val, and Ile; X₁₇ is Aib; X₂₀ is Glu; X₂₁ is selected from the group consisting of Asp and Glu; X₂₄ is Arg; X₂₇ is Glu; X₂₈ is Ser.

In some embodiments, X₃ is selected from the group consisting of His and Gln; X₁₅ is Asp; X₁₆ is selected from the group consisting of Thr, Leu, Val, and Ile; X₁₇ is Aib; X₂₀ is Gin; X₂₁ is Glu; X₂₄ is selected from the group consisting of Gln and Glu; X₂₇ is Glu; X₂₈ is Ser.

In some embodiments, R₁ is hydrogen; R₂ is -OH or -NH₂. In some embodiments, R₁ is hydrogen; R₂ is -OH.

The glucagon analog, or the pharmaceutically acceptable salt and/or solvate thereof disclosed herein, has glucagon receptor (GCGR) agonist activity, and the binding to GCGR can be used as an indicator of agonist activity. In some alternative embodiments, intracellular signal transduction resulting from the binding of the compound to the receptor may also be measured. For example, the activation of GCGR by a glucagon receptor agonist stimulates the formation of cyclic adenosine monophosphate (cAMP) in cells. Thus, the receptor activity can be monitored by cAMP production in proper cells expressing the receptor. For example, the assays can be conducted using the materials provided in Example 2 (e.g., CHO-K1/GCGR/CRE-Luc transfected cells and Cisbio-cAMP-Gs Dynamic kit) in the conditions described. EC₅₀ values can be used as a numerical measure of agonist potency for GCGR. The EC₅₀ value is the concentration of a compound required to achieve half the maximum activity of the compound for a receptor in discussion in a particular assay.

In some embodiments, the glucagon receptor is a mammalian glucagon receptor, such as a human glucagon receptor.

In some embodiments, the agonist activity of the glucagon analog, or the pharmaceutically acceptable salt and/or solvate thereof disclosed herein for human GCGR is at least 1.5 folds, at least 1.6 folds, at least 1.7 folds, at least 1.8 folds, at least 1.9 folds, at least 2.0 folds, at least 2.1 folds, at least 2.2 folds, at least 2.3 folds, at least 2.4 folds, at least 2.5 folds, at least 2.6 folds, at least 2.7 folds, at least 2.8 folds, at least 2.9 folds, at least 3.0 folds, or at least 3.1 folds greater than the native glucagon.

In some embodiments, the glucagon analog, or the pharmaceutically acceptable salt and/or solvate thereof disclosed herein, has no or negligible agonist activity for human GLP-1R and/or GIPR.

In some embodiments, the glucagon analog, or the pharmaceutically acceptable salt and/or solvate thereof disclosed herein, has increased solubility and/or stability as compared with the native glucagon.

In some embodiments, the increased solubility may include or refer to increased solubility at pH 4 (e.g., in an acetate buffer at pH 4), pH 5 (e.g., in an acetate buffer at pH 5), pH 6 (e.g., in a phosphate buffer at pH 6), pH 7 (e.g., in a phosphate buffer at pH 7), and/or pH 7.5 (e.g., in a phosphate buffer at pH 7.5) as compared with the native glucagon. As an example, the assay can be conducted in the conditions given in Example 5. A solubility > 1 mg/mL may be desired.

In some embodiments, for example, by measuring the absorbance at 280 nm and calculation according to the Beer's law, the solubility of the glucagon analog disclosed herein in a phosphate buffer at pH 7.4 is ≥ 1.00 mg/mL, ≥ 1.10 mg/mL, ≥ 1.20 mg/mL, ≥ 1.30 mg/mL, ≥ 1.40 mg/mL, ≥ 1.50 mg/mL, ≥ 1.60 mg/mL, ≥ 1.70 mg/mL, ≥ 1.80 mg/mL, ≥ 1.90 mg/mL, ≥ 2.00 mg/mL, ≥ 2.10 mg/mL, ≥ 2.20 mg/mL, ≥ 2.30 mg/mL, ≥ 2.40 mg/mL, ≥ 2.50 mg/mL, ≥ 2.60 mg/mL, ≥ 2.70 mg/mL, ≥ 2.80 mg/mL, ≥ 2.90 mg/mL, ≥ 3.00 mg/mL, ≥ 3.10 mg/mL, ≥ 3.20 mg/mL, ≥ 3.30 mg/mL, ≥ 3.40 mg/mL, ≥ 3.50 mg/mL, ≥ 3.60 mg/mL, ≥ 3.70 mg/mL, ≥ 3.80 mg/mL, ≥ 3.90 mg/mL, ≥ 4.00 mg/mL, ≥ 4.10 mg/mL, ≥ 4.20 mg/mL, ≥ 4.30 mg/mL, ≥ 4.40 mg/mL, ≥ 4.50 mg/mL, ≥ 4.60 mg/mL, ≥ 4.70 mg/mL, ≥ 4.80 mg/mL, ≥ 4.90 mg/mL, or ≥ 5.00 mg/mL.

In some embodiments, the increased stability may include or refer to increased physical stability and/or increased chemical stability as compared with the native human glucagon.

In some embodiments, the increased physical stability may include or refer to a reduced tendency to aggregate, e.g., to form soluble or insoluble aggregates (e.g., fibrils). The aggregation (e.g., fibrillation) can be determined by dissolving the glucagon analog in 0.1 N HCl at a concentration of 1 mg/mL at 37 °C. Any suitable time period may be used, for example, 24 hours, 48 hours, 96 hours, or 120 hours. The aggregation can be determined using a ThT fluorescence assay. The aggregation can be determined in the conditions given in Example 4.

In some embodiments, the increased chemical stability may include or refer to a reduced tendency of peptide cleavage or degradation in an aqueous buffer (generally in the absence of contaminating protease or peptidase activity). The stability can be determined by, for example, dissolving the glucagon analog in a phosphate buffer at pH 7.4 at a concentration of 1 mg/mL at 4 °C, 25 °C, or 40 °C. The assessment may include determining the stability after incubation for a proper period of time, e.g., 1 day, 7 days, 14 days, 21 days, or 28 days. This may include determining the purity of the compound, i.e., the percentage area of the main peak relative to the total area of all integrated peaks in the chromatograms, as defined in Example 4. The stability can be determined in the conditions given in Example 4.

### Polynucleotide

The present disclosure provides a polynucleotide, which may be RNA, DNA, or cDNA, encoding a precursor peptide of the glucagon analog disclosed herein. According to some embodiments of the present disclosure, the polynucleotide disclosed herein is a substantially isolated polynucleotide.

The precursor peptide is a peptide that can give the glucagon analog disclosed herein after modification. For example, X₁₇ in the precursor peptide is Arg or another natural amino acid, and the glucagon analog disclosed herein can be given by modifying X₁₇ to Aib.

The polynucleotide disclosed herein may be in the form of, may be present in, and/or may be part of a vector, such as a plasmid, cosmid, YAC, or viral vector. The vector may be particularly an expression vector, i.e., a vector that can provide expression of the glucagon analog *in vitro* and/or *in vivo* (i.e., in a suitable host cell, host organism, and/or expression system). The expression vector generally comprises at least one of the polynucleotides of the present disclosure, which is operably linked to one or more suitable expression regulatory elements (e.g., promoters, enhancers, terminators, and the like). The selection of the elements and their sequences for expression in a particular host is within the knowledge of those skilled in the art.

In some embodiments, the vector comprises the following elements that are operably linked in the 5'→ 3' direction: a promoter for driving the expression of the nucleic acid fragment, an optional polynucleotide encoding a leader peptide enabling the secretion (into the extracellular phase or into the periplasm as appropriate), a polynucleotide encoding a precursor peptide, and an optional polynucleotide encoding a terminator. The polynucleotide of the present disclosure may be prepared or obtained by known means (e.g., by automatic DNA synthesis and/or recombinant DNA techniques) based on information on the amino acid sequence of a precursor peptide of the glucagon analog disclosed herein.

### Host Cell

The present disclosure provides a recombinant host cell expressing a precursor peptide of the glucagon analog disclosed herein, or containing the polynucleotide or the vector disclosed herein. In some embodiments, the host cell is a bacterial cell, a fungal cell, or a mammalian cell.

Bacterial cells include, for example, cells of gram-negative bacterial strains (e.g., *Escherichia coli* strains, *Proteus* strains, and *Pseudomonas* strains), and gram-positive bacterial strains (e.g., *Bacillus* strains, *Streptomyces* strains, *Staphylococcus* strains, and *Lactococcus* strains).

Fungal cells include, for example, cells of species of *Trichoderma, Neurospora,* and *Aspergillus;* or cells of species of *Saccharomyces* (e.g., *Saccharomyces cerevisiae), Schizosaccharomyces* (e.g., *Schizosaccharomyces pombe), Pichia* (e.g., *Pichia pastoris,* and *Pichia methanolica),* and *Hansenula.*

Mammalian cells include, for example, HEK293 cells, CHO cells, BHK cells, HeLa cells, COS cells, and the like.

However, amphibian cells, insect cells, plant cells, and any other cells used in the art for expressing heterologous proteins may also be used in the present disclosure.

The cells of the present disclosure are unable to develop into a complete plant or individual animal.

### Production or Preparation Method

The present disclosure provides a method for preparing the glucagon analog disclosed herein, including, for example, the following methods:
(a) synthesizing the glucagon analog by chemical synthesis (e.g., solid-phase or liquid-phase synthesis) and recovering the synthesized glucagon analog thereby obtained; or
(b) expressing a precursor peptide from a polynucleotide or vector encoding the precursor peptide, recovering the expression product, and modifying the precursor peptide to give the glucagon analog disclosed herein, e.g., by introducing one or more unnatural amino acids (e.g., Aib).

In one embodiment, the method comprises the following steps:
- culturing the host cell disclosed herein in a condition allowing the expression of a precursor peptide;
- recovering the precursor peptide from the culture; and
- modifying the precursor peptide to give the glucagon analog disclosed herein. Optionally, the method further comprises a step of purifying the precursor peptide.

Methods and reagents for recombinant production of polypeptides, e.g., specific suitable expression vectors, transformation or transfection methods, selection labels, methods for inducing protein expression, culture conditions, and the like, are known in the art. Similarly, polypeptide isolation and purification techniques suitable for use in the methods of producing precursor peptides are well known to those skilled in the art. The methods of solid-phase or liquid-phase synthesis are known in the art. For example, reference may be made to WO98/11125, in particular to Fields, GB et al., 2002, "Principles and practice of solid-phase peptide synthesis". In: Synthetic Peptides (second edition), and examples in the present disclosure.

In some embodiments, the present disclosure further provides a method for forming a pharmaceutically acceptable salt and/or solvate of a glucagon analog. For example, the glucagon analog disclosed herein can react with a pharmaceutically acceptable acidic or basic compound to form a salt.

In some embodiments, the glucagon analog disclosed herein can be prepared by recombinant expression in microorganisms, or by Fmoc solid-phase synthesis (e.g., the SPPS method, including methods for amino acid deprotection, methods for the cleavage of peptides from resins, and methods for the purification of peptides).

In some embodiments, when Fmoc solid-phase synthesis is used, the synthetic support is Fmoc-L-Thr (tBu)-Wang resin (GL Biochem, loading capacity: 0.533 mmol/g). In some embodiments, amino acid derivatives used during synthesis include: Fmoc-L-Ala-OH, Fmoc-L-Arg (Pbf)-OH, Fmoc-L-Asn (Trt)-OH, Fmoc-L-Asp (OtBu)-OH, Fmoc-L-Gln (Trt)-OH, Fmoc-L-Glu (OtBu)-OH, Fmoc-Gly-OH, Fmoc-L-His (Trt)-OH, Fmoc-L-Ile-OH, Fmoc-L-Leu-OH, Fmoc-L-Lys (Boc)-OH, Fmoc-L-Met-OH, Fmoc-L-Phe-OH, Fmoc-L-Pro-OH, Fmoc-L-Ser (tBu)-OH, Fmoc-L-Thr (tBu)-OH, Fmoc-L-Trp (Boc)-OH, Fmoc-L-Tyr (tBu)-OH, Fmoc-L-Val-OH, Fmoc-Aib-OH, Fmoc-L-Nle-OH, and the like. In some embodiments, the synthetic process comprises: after washing the solid-phase synthesis support, the Fmoc protecting groups on the alpha-amino groups of the solid-phase synthesis support is removed by using an N,N-dimethylformamide (DMF) solution containing 20% of 4-methylpiperidine, and the solid-phase support and the next amino acid derivative in the sequence in excess amounts are activated by using HCTU/4-methylmorpholine and condensed to form an amide bond to extend the peptide chain; the procedures of condensation → washing → deprotection → washing → the next round of amino acid condensation are repeated to reach the desired length of the polypeptide chain, finally a mixed solution of trifluoroacetic acid:water:triisopropylsilane = 90:5:5 (v:v:v) is reacted with the resin to cleave the polypeptide from the solid phase carrier, and the mixture is precipitated using frozen methyl tert-butyl ether to give a solid crude product of the polypeptide derivative; the solid crude product of the polypeptide is dissolved in a mixture of acetonitrile/water containing 0.1% of trifluoroacetic acid and then purified and separated by a C-18 reverse-phase preparative chromatographic column to give the pure products of the polypeptide and the derivatives thereof.

### Pharmaceutical Composition

The present disclosure provides a pharmaceutical composition comprising a prophylactically, therapeutically, or alleviatively effective amount of the glucagon analog or the pharmaceutically acceptable salt and/or solvate thereof disclosed herein, and one or more pharmaceutically acceptable carriers, diluents, buffers or excipients. In some embodiments, the pharmaceutical composition may comprise 0.01 to 99 wt% of the glucagon analog, or the pharmaceutically acceptable salt and/or solvate thereof disclosed herein, in a unit dose. In other embodiments, the amount of the glucagon analog or the pharmaceutically acceptable salt and/or solvate thereof disclosed herein in a unit dose of the pharmaceutical composition is 0.1-2000 mg, and in some specific embodiments, 1-1000 mg.

In some embodiments, the pharmaceutical composition further comprises at least one compound or substance having therapeutic activity for a metabolic disease.

In some embodiments, the compound or substance having therapeutic activity for a metabolic disease is selected from the group consisting of one or more of the following: glucose-dependent insulinotropic polypeptide (GIP), a glucagon-like peptide-1 (GLP-1) receptor agonist, insulin, enteroglucagon, a neuropeptide Y5 receptor antagonist, an acetyl-CoA carboxylase inhibitor, a leptin receptor agonist, a glinide, an alpha-glucosidase inhibitor (AGi), a thiazolidinedione (TZD), a dipeptidyl peptidase-4 inhibitor (DPP-IV), a sodium-glucose cotransporter 2 (SGLT-2) inhibitor, a farnesol X receptor (FXR) agonist, and obestatin.

The pharmaceutical composition disclosed herein may be administered parenterally, e.g., administered subcutaneously, intramuscularly, intraperitoneally, or intravenously with a syringe (optionally, an injection pen). Alternatively, the parenteral administration may be performed using an infusion pump. An additional option is that the composition may be a solution or suspension of glucagon peptide for administration in the form of a nasal or intrapulmonary spray; alternatively, the composition may be administered transdermally, e.g., by needle-free injection or by a patch, optionally an iontophoretic patch; alternatively, the composition may be administered transmucosally (e.g., orally).

### Kit (or Kit-of-Parts)

The present disclosure further provides a kit comprising the glucagon analog or the pharmaceutically acceptable salt and/or solvate thereof disclosed herein or a pharmaceutical composition comprising same, and an administration device.

In some embodiments, the device comprises a syringe and a needle. In some specific embodiments, the device is a pre-filled syringe.

In some embodiments, the glucagon analog or the pharmaceutically acceptable salt and/or solvate thereof disclosed herein, or the pharmaceutical composition comprising same, is in a pre-filled syringe.

### Methods for Preventing or Treating Disease or Condition and Pharmaceutical Use

The present disclosure further provides use of the glucagon analog or the pharmaceutically acceptable salt and/or solvate thereof described above, and/or the pharmaceutical composition described above, in preparing a medicament for preventing or treating a disease or condition.

The present disclosure further provides the glucagon analog or the pharmaceutically acceptable salt and/or solvate thereof described above, and/or the pharmaceutical composition described above, for use in preventing or treating a disease or condition. The present disclosure further provides a method for preventing or treating a disease or condition, comprising: administering to a subject in need thereof a therapeutically effective amount of the glucagon analog or the pharmaceutically acceptable salt and/or solvate thereof disclosed herein, and/or a therapeutically effective amount of the pharmaceutical composition disclosed herein.

In some embodiments, the disease or condition is selected from the group consisting of hypoglycemia, hyperglycemia, type 2 diabetes mellitus, type 1 diabetes mellitus, coronary heart disease, atherosclerosis, beta-blocker toxicity, insulinoma, and Von Gierke disease; or the disease or condition is selected from the group consisting of impaired glucose tolerance, dyslipidemia, hypertension, overweight, binge eating, hepatic steatosis, and obesity.

In some embodiments, the hypoglycemia is selected from the group consisting of pathological hypoglycemia and non-pathological hypoglycemia.

In some embodiments, the hypoglycemia is selected from the group consisting of diabetic hypoglycemia, non-diabetic hypoglycemia, fasting hypoglycemia, drug-induced hypoglycemia, acute insulin-induced hypoglycemia, gastric bypass-induced hypoglycemia, alcohol-induced hypoglycemia, reactive hypoglycemia, and gestational hypoglycemia.

In some embodiments, the present disclosure provides a method for rapidly increasing a glucose level, restoring a normal blood glucose level, stabilizing a blood glucose level, or preventing or treating hypoglycemia, comprising: administering to a subject in need an effective amount of the glucagon analog or the pharmaceutically acceptable salt and/or solvate thereof disclosed herein, or a pharmaceutical composition comprising same.

In some embodiments, the present disclosure provides a method for reducing weight gain or causing weight loss, comprising: administering to a subject in need an effective amount of the glucagon analog or the pharmaceutically acceptable salt and/or solvate thereof disclosed herein, or a pharmaceutical composition comprising same. The method for reducing weight gain or causing weight loss is expected to be useful in treating obesity of various causes, including drug-induced obesity, and in reducing complications associated with obesity, including vascular diseases (e.g., coronary artery disease, stroke, peripheral vascular disease, ischemic reperfusion, etc.), hypertension, type II diabetes mellitus, hyperlipidemia, and skeletal muscle diseases. The present disclosure provides a pharmaceutical use of the glucagon analog or the pharmaceutically acceptable salt and/or solvate thereof, or the pharmaceutical composition comprising same in preparing a medicament for preventing, treating, or alleviating obesity.

In some embodiments, the present disclosure provides a method for treating hyperglycemia or diabetes, comprising: administering the glucagon analog or the pharmaceutically acceptable salt and/or solvate thereof disclosed herein, or a pharmaceutical composition comprising same, in combination with insulin. The combination of insulin (e.g., basal, rapid-acting, or long-acting insulin) with the glucagon analog or the pharmaceutically acceptable salt and/or solvate thereof disclosed herein or a pharmaceutical composition comprising same can reduce nocturnal hypoglycemia and/or buffer the hypoglycemic effects of insulin. The present disclosure provides a pharmaceutical use of the glucagon analog or the pharmaceutically acceptable salt and/or solvate thereof, or the pharmaceutical composition comprising same in preparing a medicament for preventing, treating, or alleviating hyperglycemia or diabetes.

In some embodiments, the method described above is for a human subject, and the pharmaceutical use is for use of a medicament in a human subject.

### BRIEF DESCRIPTION OF THE DRAWINGS

FIG. 1 illustrates the results of assessing the physical stability of the glucagon analog by ThT fibrillogenesis assay.
FIG. 2 illustrates the efficacy results of glucagon analog 40 and Dasiglucagon in a normoglycemic rat model.
FIG. 3 illustrates the efficacy results of glucagon analog 40 and Dasiglucagon in a hypoglycemic rat model.
FIG. 4A illustrates the results of the hemolysis assay of Dasiglucagon; FIG. 4B illustrates the results of the hemolysis assay of glucagon analog 40.
FIGs. 5A and 5B illustrate the stability results of glucagon analog 40 and Dasiglucagon in human liver microsomes and human kidney microsomes.
FIG. 6 illustrates the concentration-time curves of glucagon analog 40 and Dasiglucagon after subcutaneous or intravenous administration.

### DETAILED DESCRIPTION

### Definitions

In order to facilitate the understanding of the present disclosure, some technical and scientific terms are specifically defined below. Unless otherwise specifically defined herein, all other technical and scientific terms used herein have the meanings generally understood by those of ordinary skills in the art to which the present disclosure belongs.

The term "glucagon analog" refers to a compound having at least a part of, all of, or more agonist activity of the native glucagon for a glucagon receptor. For example, the glucagon analog includes, but is not limited to: a polypeptide comprising any amino acid substitution, insertion or deletion, or a post-translational modification or other chemical modification on the basis of an amino acid sequence set forth in SEQ ID NO: 51, provided that the analog has activity in stimulating the glucagon receptor, e.g., as determined by cAMP production according to the method described in Example 2.

In the present disclosure, the glucagon analog also includes peptides with a modified amino terminus and/or carboxy terminus, for example, a peptide comprising an amido group in place of a terminal carboxylic acid. For example, the glucagon analog disclosed herein may comprise an "H-" moiety introduced at the amino terminus (N terminus) of the sequence, and an "-OH" moiety or an "-NH₂" moiety at the carboxy terminus (C terminus) of the sequence. In this case, unless otherwise specified, the "H-" moiety at the N terminus of the sequence in discussion represents a hydrogen atom [i.e., in formula (I), R₁ = hydrogen = H, corresponding to the presence of a free primary or secondary amino group at the N terminus], and the "-OH" or "-NH₂" moiety at the C terminus of the sequence represents a hydroxyl group [e.g., in formula (I), R₂ = OH, corresponding to the presence of a carboxyl group (COOH) at the C terminus] or an amino group [e.g., in formula (I), R₂ = NH₂, corresponding to the presence of an amido group (CONH₂) at the C terminus]. In each sequence of the present disclosure, the C-terminal "-OH" moiety may be replaced by the C-terminal "-NH₂", and vice versa.

The "native glucagon" refers to the native human glucagon having the sequence: 
H-His-Ser-Gln-Gly-Thr-Phe-Thr-Ser-Asp-Tyr-Ser-Lys-Tyr-Leu-Asp-Ser-Arg-Arg-Ala-
Gln-Asp-Phe-Val-Gln-Trp-Leu-Met-Asn-Thr-OH (SEQ ID NO: 51).
 "Dasiglucagon" refers specifically to a glucagon analog developed by Zealand Pharma having the amino acid sequence: H-HSQGTFTSDYSKYLDAibARAEEFVKWLEST-OH (SEQ ID NO: 52).

In the present disclosure, the term "GLP-1" refers to human GLP-1 (7-36 or 7-37), unless otherwise stated; the term "GIP" refers to human GIP (1-42).

The three-letter and single-letter codes for amino acids used herein are as described in J. biol. chem., 243, p3558 (1968). Unless otherwise specified, all amino acid residues in the present disclosure are in the L-configuration. Furthermore, Aib is 2-aminoisobutyric acid (also known as alpha-aminoisobutyric acid), alpha-methyl-Ser (alpha-methylserine) is a serine substituted with a methyl group at the alpha-position H, and pGlu is pyroglutamic acid.

The term "natural amino acids" refers to 20 conventional amino acids, i.e., alanine (A), cysteine (C), aspartic acid (D), glutamic acid (E), phenylalanine (F), glycine (G), histidine (H), isoleucine (I), lysine (K), leucine (L), methionine (M), asparagine (N), proline (P), glutamine (Q), arginine (R), serine (S), threonine (T), valine (V), tryptophan (W), and tyrosine (Y).

The term "unnatural amino acids" refers to amino acids that are not naturally encoded, or are not found in the genetic code of any organism. They may be, for example, chemically synthesized compounds. Examples of the unnatural amino acids include, but are not limited to, hydroxyproline, γ-carboxyglutamic acid, O-serine phosphate, azetidinecarboxylic acid, 2-aminoadipic acid, 3-aminoadipic acid, beta-alanine, aminopropionic acid, 2-aminobutyric acid, 4-aminobutyric acid, 6-aminohexanoic acid, 2-aminoheptanoic acid, 2-aminoisobutyric acid (Aib), 3-aminoisobutyric acid, 2-aminopimelic acid, *t*-butylglycine, 2,4-diaminoisobutyric acid (Dap), desmosine, 2,2'-diaminopimelic acid, 2,3-diaminopropionic acid (Dab), N-ethylglycine, *N*-methylglycine, *N*-ethylasparagine, homoproline, hydroxylysine, allo-hydroxylysine, 3-hydroxyproline, 4-hydroxyproline, isodesmosine, allo-isoleucine, *N*-methylalanine, *N*-methylglycine, *N*-methylisoleucine, *N*-methylpentylglycine, *N*-methylvaline, naphthalanine, norvaline, norleucine (Nle), omithine (Orn), D-ornithine, D-arginine, p-aminophenylalanine, pentylglycine, pipecolic acid and thioproline. Furthermore, the term "unnatural amino acid" also includes compounds obtained by chemical modification of the C-terminal carboxyl group, N-terminal amino group and/or side chain functional group of a natural amino acid or an unnatural amino acid.

The term "agonist" is defined as a substance or ligand that activates the receptors in discussion. For example, the term GLP-1 or GIP or glucagon (receptor) agonist as used in the context of the present disclosure refers to a substance or ligand that can activate a GLP-1 receptor or GIP receptor or glucagon receptor.

The term "pharmaceutically acceptable salt" refers to a salt that is not deleterious to the patient or subject to be treated. Generally, such a salt is an acid addition or base addition salt.

The term "solvate" refers to a complex formed from a solute (in this case, the glucagon analog or the pharmaceutically acceptable salt thereof disclosed herein) and a solvent in stoichiometrically defined amounts.

The term "pharmaceutical composition" refers to a mixture containing one or more of the glucagon analog or the physiologically/pharmaceutically acceptable salt or prodrug thereof described herein, and other chemical components, for example, a physiologically/pharmaceutically acceptable carrier and excipient.

The term "pharmaceutically acceptable carrier" includes any standard pharmaceutical carrier or diluent, such as those used in compositions or formulations suitable for oral, pulmonary, rectal, nasal, topical, subcutaneous, intramuscular, intravenous, intraperitoneal, intradermal, transdermal, or intravaginal administration.

The term "treatment" refers to a method for acquiring a beneficial or desired clinical result. For the purposes of the present disclosure, the beneficial or desired clinical result includes, but is not limited to, alleviation of a condition, reduced severity of a disease, stabilized (i.e., non-deteriorating) state of a disease, delayed or alleviated disease progression, ameliorated or alleviated disease state, and (partial or complete) remission, whether detectable or undetectable. The "treatment" may also refer to prolonging the survival as compared to the expected survival without the treatment. The "treatment" is an intervention performed with the aim of preventing the onset of a disease or altering the pathological condition of a disease. Thus, the "treatment" refers to both therapeutic treatments and prophylactic or preventative measures. As used in the context for prophylactic or preventative measures, the compound is not necessarily a complete prevention of the disease or condition. Subjects in need of the treatment include subjects already suffering from the disease or in need of prevention of the onset of the disease. The "treating" also refers to inhibiting or reducing an increase in a pathological condition or symptom (e.g., weight gain or hypoglycemia) as compared to a case where no treatment is provided, and does not imply a complete cessation of the associated condition.

The term "effective amount" or "therapeutically effective amount" refers to an amount or dose sufficient to cure, alleviate, or partially arrest, or otherwise promote the cure or recovery of a given condition (disorder, disease) or injury and preferably the complications that result therefrom. The amount or dose effective for a particular purpose depends on the severity of the disease or condition and the weight and general condition of the subject or patient to be treated. The determination of an appropriate amount or dose is within the capability of a trained physician (or veterinarian) of ordinary skills.

The term "subject" is used interchangeably with "patient", "individual", "object", and the like, and refers to a human or non-human animal, including, but not limited to, mammals, such as human, primates, livestock animals (e.g., cows, pigs), pets (e.g., dogs, cats), and rodents (e.g., mice and rats).

### Examples

The present disclosure is further illustrated by the following examples, which, however, are not intended to limit the present disclosure. The experimental methods in the examples of the present disclosure in which specific conditions are not specified are generally performed under conventional conditions such as a regular Fmoc solid-phase polypeptide synthesis manual, or Antibodies: A Laboratory Manual and Molecular Cloning: A Laboratory Manual by Cold Spring Harbor Laboratory, or under conditions recommended by the manufacturer of the raw material or the goods. Reagents without specified origins are commercially available conventional reagents.

The major reagents (sources) of the present disclosure are as follows: Fmoc-Thr (tBu)-Wang resin (GL Biochem), HCTU (High fine Biotech), Fmoc-Aib-OH (GL Biochem), N,N-dimethylformamide (Sinopharm), dichloromethane (Sinopharm), trifluoroacetic acid (TCI Chemicals), triisopropylsilane (TCI Chemicals), acetonitrile (Merck-Millipore), diisopropylethylamine (Sigma), 4-methylpiperidine (TCI Chemicals), methyl tert-butyl ether (TCI Chemicals), 4-methylmorpholine (TCI Chemicals), Fmoc-Nle-OH (GL Biochem). DMEM/F12 (Gibco 11330032), Casein, 3-isobutyl-1-methylxanthine (Sigma I7018-250MG), cAMP-Gs Dynamic kit (Cisbio 62AM4PEC, 20000 tests), 384-well plates (Sigma CLS4514-50EA), 96-well V-bottom plates (PS; Axygen WIPP02280), 96-well plates (Cisbio 66PL96100), Countess^{®} Cell Counting Chamber Slides (Invitrogen C10228), Cisbio-cAMP-Gs Dynamic kit (Cisbio 62AM4PEC), 0.25% trypsin-EDTA and Phenol Red (ThermoFisher 25200-114), fetal bovine serum (Gibco^{™}, ThermoFisher 10099-141), and human glucagon (Abmole M9312).

The major instruments (sources) of the present disclosure are as follows: H-CLASS analytical UPLC system (WATERS), Agilent 1290-6530 ultra-high-performance liquid chromatography-mass spectrometry (Agilent Technologies), LC Prep-150 system (WATERS), Prelude-X system (Protein Technologies), microplate reader (BioTek Hl MFD; Tecan-Infinite F Plex).

### Example 1. Synthesis of glucagon analogs

The compounds in Table 1 were designed and synthesized according to the following method in this example.

**Table 1. Glucagon analogs (or, compounds or compounds disclosed herein)**

| Compound No. | Compound sequences | SEQ ID NO: |
|---|---|---|
| 1 | H-HSQGTFTSDYSKAibLDSRRAQDFVQWLMNT-OH | 1 |
| 2 | H-HSQGTFTSDYSKYLD(alpha-methyl-Ser)RRAQDFVQWLMNT-O H | 2 |
| 3 | H-HSQGTFTSDYSKYLDSAibRAQDFVQWLMNT-OH | 3 |
| 4 | H-HSQGTFTSDYSKYLDSRAibAQDFVQWLMNTOH | 4 |
| 5 | H-HSQGTFTSDYSKYLDSRRAQDFV(alpha-methyl-Ser)WLMNT-O H | 5 |
| 6 | H-HSQGTFTSDYSKYLESRAibAEDFVQWLMNT-OH | 6 |
| 7 | H-HSQGTFTSDYSKYLESRAibAEDFVQWLLET-OH | 7 |
| 8 | H-HSQGTFTSDYSKYLESRAibAQEFVEWLMNT-OH | 8 |
| 9 | H-HSQGTFTSDYSKYLESRAibAQEFVEWLLST-OH | 9 |
| 10 | H-HSQGTFTSDYSKYLESRAibAQEFVSWLLET-OH | 10 |
| 11 | H-HSQGTFTSDYSKYLDSRAibAEEFVSWLLST-OH | 11 |
| 12 | H-HSQGTFTSDYSKYLDTRAibAEEFVSWLSET-OH | 12 |
| 13 | H-HSQGTFTSDYSKYLESAibRAQEFVQWLMNT-OH | 13 |
| 14 | H-HSQGTFTSDYSKYLDSAibRAQDFVQWLLST-OH | 14 |
| 15 | H-HSQGTFTSDYSKYLDSAibRAQDFVQWLEST-OH | 15 |
| 16 | H-HSQGTFTSDYSKYLDSAibRAQDFVQWLLET-OH | 16 |
| 17 | H-HSQGTFTSDYSKYLESAibRAQEFVQWLLST-OH | 17 |
| 18 | H-HSQGTFTSDYSKYLESAibRAQEFVQWLEST-OH | 18 |
| 19 | H-HSQGTFTSDYSKYLESAibRAQEFVQWLLET-OH | 19 |
| 20 | H-HSQGTFTSDYSICYLESAibRAQEFVEWLLST-OH | 20 |
| 21 | H-HSQGTFTSDYSKYLESAibRAQEFVEWLEST-OH | 21 |
| 22 | H-HSQGTFTSDYSKYLESAibRAQEFVEWLLET-OH | 22 |
| 23 | H-HSQGTFTSDYSKYLESRAibAQEFVEWLEST-OH | 23 |
| 24 | H-HSQGTFTSDYSKYLESRAibAQEFVEWLLET-OH | 24 |
| 25 | H-HSQGTFTSDYSKYLESKAibAQEFVEWLEST-OH | 25 |
| 26 | H-HSQGTFTSDYSKYLESEAibAQEFVEWLEST-OH | 26 |
| 27 | H-HSQGTFTSDYSKYLESAibRAQDFVQWLLST-OH | 27 |
| 28 | H-HSQGTFTSDYSKYLESAibRAQDFVQWLEST-OH | 28 |
| 29 | H-HSQGTFTSDYSKYLESAibRAQDFVQWLLET-OH | 29 |
| 30 | H-HSQGTFTSDYSKYLESAibRAQDFVQWLEET-OH | 30 |
| 31 | H-HSQGTFTSDYSKYLESAibRAQEFVQWLEET-OH | 31 |
| 32 | H-HSQGTFTSDYSKYLESAibRAQDFVEWLLST-OH | 32 |
| 33 | H-HSQGTFTSDYSKYLESAibRAQDFVEWLEST-OH | 33 |
| 34 | H-HSQGTFTSDYSKYLESAibRAQDFVEWLLET-OH | 34 |
| 35 | H-HSQGTFTSDYSKYLESAibRAQDFVEWLEET-OH | 35 |
| 36 | H-HSQGTFTSDYSKYLESAibRAQEFVEWLEET-OH | 36 |
| 37 | H-HSQGTFTSDYSKYLESAibRAKDFVEWLEST-OH | 37 |
| 38 | H-HSQGTFTSDYSKYLESAibRAEDFVRWLEST-OH | 38 |
| 39 | H-HSQGTFTSDYSKYLDTAibRAQEFVQWLEST-OH | 39 |
| 40 | H-HSQGTFTSDYSKYLDLAibRAQEFVQWLEST-OH | 40 |
| 41 | H-HSQGTFTSDYSKYLDVAibRAQEFVQWLEST-OH | 41 |
| 42 | H-HSQGTFTSDYSKYLDIAibRAQEFVQWLEST-OH | 42 |
| 43 | H-HSHGTFTSDYSKYLDLAibRAQEFVQWLEST-OH | 43 |
| 44 | H-HSQGTFTSDYSKYLDLAibRAEDFVRWLEST-OH | 44 |
| 45 | H-HSQGTFTSDYSKYLDLAibRAEDFVQWLEST-OH | 45 |
| 46 | H-HSHGTFTSDYSKYLDLAibRAEEFVQWLEST-OH | 46 |
| 47 | H-HSHGTFTSDYSKYLDLAibRAQEFVEWLEST-OH | 47 |
| 48 | H-HSQGTFTSDYSKYLDLAibRAEEFVRWLEST-OH | 48 |
| 49 | H-HSQGTFTSDYSKYLDLAibRAEEFVQWLEST-OH | 49 |

### 1. Chemical synthesis of compound 1

### 1.1 Synthesis of polypeptide skeleton

0.1 mmol of solid-phase synthesis support Fmoc-Thr (tBu)-Wang resin was transferred into a disposable polypropylene reaction tube for solid-phase polypeptide synthesis. DMF (10 mL) was added to swell the resin for 10 minutes and removed at reduced pressure. The resin was then washed with DMF (10 mL) twice. The synthesis of the polypeptide skeleton was completed using a Prelude-X system. A 10-fold excess of the corresponding Fmoc-protected amino acid was used for amide bond condensation, and the amino acid condensation was performed by reaction at room temperature for 30 minutes after activation with HCTU/4-methylmorpholine. The removal of the Fmoc protecting group was conducted by 2 reactions at room temperature in a 20% solution of 4-methylpiperidine in N,N-dimethylformamide, each for 10 min. In addition, for the condensation of the N-terminal amino acid beside Aib, two or three condensation reactions were conducted at room temperature, each for 30-60 minutes, which is very important for improving the purity of the crude peptide.

### 1.2 Cleavage of resin-peptide

The resin-peptide obtained in the above step was sequentially washed with DMF and DCM 3 times and dried in vacuum. 10 mL of a freshly prepared lysate (trifluoroacetic acid:triisopropylsilane:water = 90:5:5, v:v:v) was added, and the mixture was reacted at room temperature for 3 hours with shaking. After the reaction was completed, the mixture was filtered and the resin was washed twice with trifluoroacetic acid. The filtrates were combined before a large amount of frozen methyl tert-butyl ether was added to precipitate a solid. The mixture was centrifuged and the supernatant was discarded to obtain a crude polypeptide of compound 1.

1.3 Purification of crude peptide by reverse-phase liquid chromatography The crude peptide was dissolved in a mixed solvent containing 0.1% (v/v) trifluoroacetic acid and 20% (v/v) acetonitrile/water. The solution was filtered through a 0.22 µm membrane. The filtrate was separated using a WATERS Prep150 LC reverse-phase high-performance liquid chromatography system with mobile phases A (0.1% trifluoroacetic acid and 10% acetonitrile in water, v/v) and B (0.1 % trifluoroacetic acid and 90% acetonitrile in water, v/v). The chromatographic column was an X-SELECT OBD C-18 reverse-phase chromatographic column (WATERS), and in the purification process, the detection wavelength of the chromatograph was set as 220 nm, and the flow rate was 20 mL/min. The related fractions of the product were collected and freeze-dried to obtain a pure polypeptide product of compound 1, with a yield of 20%. The purity and the compound identity of the pure polypeptide product were determined by analytical high-performance liquid chromatography and high-performance liquid chromatography/mass spectrometry, wherein the purity was 96.09%, and the molecular weight was correct.

### 2. Chemical synthesis of compounds 2-49

The polypeptide compounds 2-49 of the present disclosure were synthesized using the protocol for compound 1 and analyzed for purity and molecular weight using analytical UPLC and LC/MS, wherein the compound molecular weights were correct and the purities are shown in Table 2.

**Table 2. Purity of compounds**

| Compound No. | Purity (%) | | Compound No. | Purity (%) | | Compound No. | Purity (%) |
|---|---|---|---|---|---|---|---|
| 1 | 96.09 | | 18 | 93.59 | | 35 | 97.04 |
| 2 | 95.10 | | 19 | 98.19 | | 36 | 94.65 |
| 3 | 96.91 | | 20 | 96.48 | | 37 | 93.23 |
| 4 | 96.21 | | 21 | 90.85 | | 38 | 88.72 |
| 5 | 90.10 | | 22 | 92.94 | | 39 | 91.64 |
| 6 | 97.72 | | 23 | 92.28 | | 40 | 96.42 |
| 7 | 96.12 | | 24 | 97.91 | | 41 | 86.97 |
| 8 | 97.87 | | 25 | 93.19 | | 42 | 95.81 |
| 9 | 96.83 | | 26 | 96.65 | | 43 | 98.23 |
| 10 | 89.52 | | 27 | 88.63 | | 44 | 97.84 |
| 11 | 96.83 | | 28 | 91.71 | | 45 | 97.05 |
| 12 | 96.27 | | 29 | 97.01 | | 46 | 90.66 |
| 13 | 96.28 | | 30 | 96.10 | | 47 | 91.59 |
| 14 | 96.43 | | 31 | 96.02 | | 48 | 93.54 |
| 15 | 96.32 | | 32 | 92.41 | | 49 | 97.19 |
| 16 | 94.77 | | 33 | 91.78 | | / | / |
| 17 | 95.31 | | 34 | 93.40 | | / | / |

The biological assessments of the glucagon analogs synthesized in Example 1 were conducted in the following examples.

### Example 2. Agonist activity assessment of glucagon analog for glucagon receptor (GCGR), glucagon-like peptide-1 receptor (GLP-1R), and glucose-dependent insulinotropic polypeptide receptor (GIPR)

### 1. Objective

The agonist activity of the glucagon analog disclosed herein for human glucagon receptor (GCGR), glucagon-like peptide-1 receptor (GLP-1R), and glucose-dependent insulinotropic polypeptide receptor (GIPR) was determined. The activation of GCGR by related compounds will stimulate the formation of cyclic adenosine monophosphate (cAMP) in cells. Thus, the receptor activity can be monitored by cAMP production in proper cells expressing the receptor.

### 2. Procedures

Agonist activity assay for GCGR: Cryopreserved stable CHO-K1/GCGR/CRE-Luc cells were taken from a liquid nitrogen tank, thawed in a 37 °C water bath, resuspended in DMEM/F12 medium (Sigma Cat# D8437), centrifuged, and seeded into T75 culture flasks. The cells were passaged at least once for subsequent tests. Before testing, the cells were trypsinized, centrifuged, washed once, and resuspended in serum-free DMEM/F12 medium. The cell density was adjusted, and the cells were plated in a 96-well plate at a density of 2,500 cells/5 µL/well. A 1 × buffer solution in Cisbio-cAMP-Gs Dynamic kit was prepared, and IBMX was added at a final concentration of 0.5 mM. 5 µL of polypeptide serially diluted by buffer was added into each well, and the plate was shaken gently for mixing and incubated for 30 minutes at 37 °C. cAMP-d2 and Anti-cAMP-Eu³⁺-Cryptate were 20-fold diluted with cAMP Lysis and detection buffer respectively and mixed well. 5 µL of diluted cAMP-d2 solution and 5 µL of diluted Anti-cAMP-Eu³⁺-Cryptate solution were sequentially added into each well. The mixture was mixed well by moderate vibration, and incubated for 1 hour at room temperature away from light.

Agonist activity assay for GLP-1R: The method differed from the method described above for the agonist activity assay for GCGR in that a stable CHO-GLP-1R cell line was used.

Agonist activity assay for GIPR: The method differed from the method described above for the agonist activity assay for GCGR in that a stable CHO-K1/GIPR cell line was used, and the cell density was 3,000 cells/5 µL/well.

### 3. Results

HTRF signal was determined using a Tecan-Infinite F Plex microplate reader with an excitation wavelength of 320 nm and emission wavelengths of 620 nm and 665 nm. The signal ratio (665 nm/620 nm × 10,000) was calculated and a non-linear fitting with the sample concentration was performed using a four-parameter equation to give the EC₅₀ values, as shown in Tables 3 and 4 below.

**Table 3. Agonist activity of the compounds for human GCGR**

| Compound No. | Human GCGR receptor EC₅₀ (nM) | Relative activity (%) |
|---|---|---|
| Dasiglucagon | 0.14 | 142 |
| 39 | 0.072 | 268 |
| 40 | 0.060 | 318 |
| 41 | 0.058 | 333 |
| 42 | 0.093 | 207 |
| 43 | 0.052 | 371 |
| 47 | 0.076 | 253 |

| | | |
|---|---|---|
| (relative activity is the activity normalized by the activity of the native glucagon) | | |

**Table 4. Agonist activity of the compounds for human GLP-1R and GIPR**

| Compound No. | GLP-1R | | GIPR | |
|---|---|---|---|---|
| | EC₅₀ (nM) | % Activity relative to native GLP-1 | EC₅₀ (nM) | % Activity relative to native GIP |
| Glucagon | 14.37 | 1.87 | >100.0 | <0.0700 |
| GLP-1 | 0.2684 | 100 | n.d. | n.d. |
| GIP | n.d. | n.d. | 0.06523 | 100 |
| 39 | 126.8 | 0.212 | > 100.0 | <0.0700 |
| 40 | 139.8 | 0.192 | > 100.0 | <0.0700 |
| 41 | 116.2 | 0.231 | > 100.0 | <0.0700 |
| 42 | 92.10 | 0.291 | > 100.0 | <0.0700 |

| | | | | |
|---|---|---|---|---|
| (n.d. indicates not detected) | | | | |

The results in Table 3 show that the agonist activity of the glucagon analogs disclosed herein for human GCGR is significantly superior to Dasiglucagon. The results in Table 4 show that the agonist activity of the glucagon analogs disclosed herein for GLP-1R and GIPR is very low and negligible as compared to those of the native glucagon, i.e., the glucagon analogs disclosed herein have good specificity for GCGR.

### Example 3. Solubility assessment of glucagon analogs in physiological condition buffer

### 1. Objective

Direct use of the native glucagon as a therapeutic agent was restricted since its solubility at neutral pH 7.4 is far below 1 mg/mL. The objective of this example was to assess the solubility of the glucagon analog disclosed herein in buffers at neutral pH, with the expectation of higher solubility as compared to the native glucagon.

### 2. Procedures

Saturated solutions of some of the glucagon analogs disclosed herein were prepared in a phosphate buffer pH 7.4. The mixture was sonicated at room temperature for 5 min and centrifuged (10000 g, 5 min). The supernatant was collected while ensuring that no visible precipitate was observed. The absorbance of the solution at 280 nm (Abs_{λ = 280 nm}) was measured, molar absorption coefficient (ε = 8250 M⁻¹cm⁻¹) of the polypeptide at 280 nm was calculated based on the contents of tryptophan and tyrosine in the sequence of the glucagon analogs, and the maximum solubility of each glucagon analog under test in neutral pH buffer was calculated according to Beer's law.

### 3. Results

The various glucagon analogs disclosed herein have significantly improved solubility over the native glucagon, as shown in Table 5. Comparing compounds 2 to 5, it was found that compound 3, which contains Aib at position 17, has increased solubility over compound 4 containing Aib at position 18; compound 3, which contains Aib at position 17, has increased solubility over compounds 2 and 4 containing alpha-methyl-Ser at position 16 or 19, respectively. It can be seen that the introduction of Aib at position 17 in the glucagon analog has an important effect on increasing the solubility of the polypeptide.

**Table 5. Solubility data of compounds**

| Compound No. | Maximum solubility (mg/mL) |
|---|---|
| Dasiglucagon | >3.75 |
| 2 | 1.27 |
| 3 | > 2.98 |
| 4 | 1.40 |
| 5 | 0.872 |
| 38 | >16.4 |
| 39 | >4.79 |
| 40 | 5.19 |
| 41 | 2.56 |
| 42 | >2.49 |
| 43 | 1.62 |
| 47 | >2.07 |

### Example 4. Physical and chemical stability assessment for glucagon analogs

The native glucagon has very poor physical and chemical stability in solutions. Aqueous solutions of the native glucagon may form gels or fibrous precipitates in hours to days and are prone to gelation in the presence of high polypeptide concentrations, external perturbations, and salts. Furthermore, the chemical instability of the native glucagon is mainly due to its sequence isomerization (aspartic acid residue), deamidation (asparagine and glutamine residue), and oxidation (methionine residue).

### 1. Objective

The purpose of this example was to assess the physical and chemical stability of the glucagon analogs disclosed herein in solutions.

### 2. Procedures for physical stability assessment

The experiment monitored the fibrillation by Thioflavin-T (ThT) fluorescence assay. Lyophilized glucagon analogs were dissolved in 0.1 N aqueous HCl at a concentration of 1 mg/mL. The polypeptide solutions were incubated on a thermostat shaker for 120 h (37 °C, 300 rpm) and the fibril content of the solutions was measured at 24 h, 48 h, and 120 h. In the ThT fluorescence assay, 8 mg of ThT powder was accurately weighed and dissolved in 50 mL of PBS buffer (1 ×). The mixture was filtered through a 0.22 µm filter membrane and stored at 4 °C away from light for later use. Before use, PBS buffer (1 ×) was 1:500 diluted to prepare a working solution. 100 µL of the working solution and 5 µL of the polypeptide solution to be detected were added into each well of a 96-well plate. The mixture was mixed well and incubated at room temperature for 20-30 min. ThT fluorescent signals were determined by a Tecan-Infinite F Plex microplate reader with an excitation wavelength of 450 nm and an emission wavelength of 482 nm. Four biological replicates were analyzed for each polypeptide.

### 3. Procedures for chemical stability assessment

Lyophilized glucagon analog powders were dissolved in a phosphate buffer (10 mM sodium dihydrogen phosphate, 15 mg/mL propylene glycol, 0.01% Tween-80, pH 7.4) at a final concentration of 1 mg/mL (determined by measuring the absorbance of the solution at 280 nm), and the sample was transferred to a glass bottle and incubated at 4 °C and 25 °C for 30 days. The purity and degradation products were detected on days 0, 7, 14, 21, and 28 after sampling by ultra-performance liquid chromatography using a C-18 reverse-phase chromatographic column, UV = 214 nm. The compound purity was calculated by the percentage area of the main peak in the chromatograms relative to the total area of all integrated peaks, normalized by the purity of the sample on day 0 for the purity.

### 4. Results

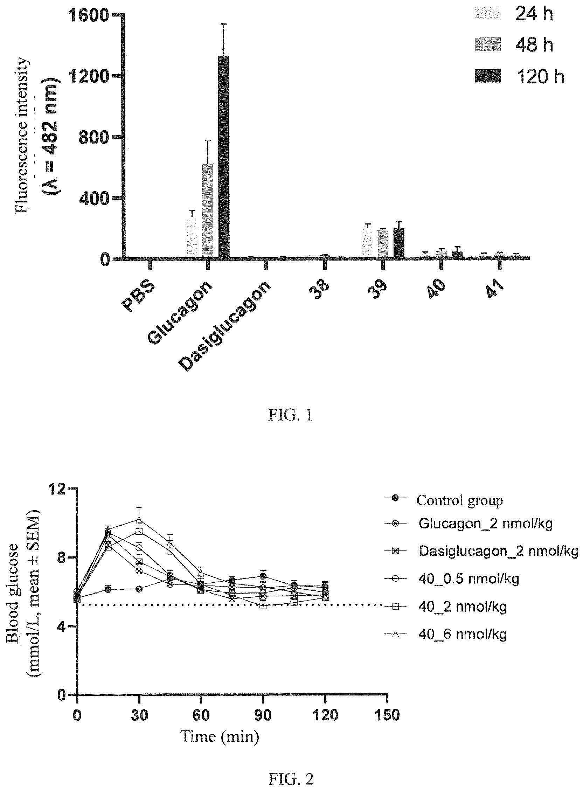

By the above procedures, the physical stability of some of the glucagon analogs disclosed herein in aqueous solution is shown in FIG. 1, and the chemical stability in phosphate buffer is shown in Table 6.

**Table 6. Chemical stability of compounds in phosphate buffer**

| Compound No. | Temperature | Purity (%) | | | | |
|---|---|---|---|---|---|---|
| | | Day 0 | Day 7 | Day 14 | Day 21 | Day 28 |
| Glucagon | 4 °C | 100.00 | n.d. | n.d. | n.d. | n.d. |
| | 25 °C | | 85.80 (precipitate) | 83.72 | 87.61 | 80.61 |
| Dasiglucagon | 4 °C | 100.00 | 98.66 | 98.84 | 99.85 | 99.68 |
| | 25 °C | | 98.07 | 96.73 | 96.68 | 96.81 |
| 39 | 4 °C | 100.00 | 97.24 | 97.45 | 95.71 | 94.88 |
| | 25 °C | | 94.64 | 93.86 | 91.35 | 91.85 |
| 40 | 4 °C | 100.00 | 99.40 | 99.87 | 100.27 | 100.59 |
| | 25 °C | | 99.70 | 99.54 | 97.63 | 97.90 |
| 41 | 4 °C | 100.00 | 98.77 | 93.35 | 89.45 | 89.12 |
| | 25 °C | | 82.25 | 68.13 | 49.04 | 33.47 |

| | | | | | | |
|---|---|---|---|---|---|---|
| (n.d. indicates that the purity detection was not available due to polypeptide precipitation.) | | | | | | |

It was found that the glucagon analogs disclosed herein exhibit superior physical and chemical stability as compared with the native glucagon, wherein compound 40 has better chemical stability in phosphate buffers than that of Dasiglucagon.

### Example 5. Efficacy assessment of glucagon analogs in normoglycemic rat model

### 1. Objective

This example was a comparative assessment of the hyperglycemic efficacy of glucagon analog 40 of the present invention, the native glucagon, and Dasiglucagon in a normal rat model.

### 2. Procedures

Male SD rats (Sprague-Dawley rats) aged 7-8 weeks were acclimatized for one week. The rats were grouped according to the body weight to ensure similar average body weights and standard deviations among groups. The animals were deprived of food 4 hours before and during the experiment, but were provided with free access to water. After 4 hours of fasting, the rats in the treatment groups were subcutaneously administered with 0.5 nmol/kg, 2 nmol/kg or 6 nmol/kg of glucagon analog 40, 2 nmol/kg of the native glucagon, or 2 nmol/kg of Dasiglucagon in a single dose, and the rats in the control group were subcutaneously administered with the same phosphate buffer as for the glucagon analog. The blood glucose was measured at T0 point by tail blood sampling before administration, and was measured at 15 min, 30 min, 45 min, 60 min, 75 min, 90 min, 105 min, and 120 min.

### 3. Results

Glucagon analog 40 raised the blood glucose level in rats in a dose-dependent manner, and exhibited short-acting characteristics similar to those of the native glucagon and Dasiglucagon. However, glucagon analog 40, at the same dose, showed a higher hyperglycemic effect. The specific results are shown in FIG. 2 and Table 7.

**Table 7. Hyperglycemic data of compounds in normal rats**

| Compounds (dose) | Blood glucose (mmol/L) | | | |
|---|---|---|---|---|
| | 15 min | 30 min | 45 min | 60 min |
| Normal saline | 6.12 | 6.15 | 6.75 | 6.42 |
| Glucagon (2 nmol/kg) | 8.72 | 7.20 | 6.42 | 6.35 |
| Dasiglucagon (2 nmol/kg) | 9.32 | 7.73 | 6.87 | 6.08 |
| 40 (0.5 nmol/kg) | 9.45 | 8.55 | 6.93 | 6.10 |
| 40 (2 nmol/kg) | 8.60 | 9.52 | 8.35 | 6.40 |
| 40 (6 nmol/kg) | 9.63 | 10.22 | 8.83 | 7.10 |

### 4. Conclusion

The results show that glucagon analog 40, the native glucagon, and Dasiglucagon are all capable of rapidly increasing blood glucose levels in rats after subcutaneous administration. Glucagon analog 40 at the dose of 0.5 nmol/kg exhibits similar hyperglycemic efficacy to Dasiglucagon at the dose of 2 nmol/kg, while at the same dose (2 nmol/kg), the efficacy of glucagon analog 40 is significantly better than that of Dasiglucagon.

In the normoglycemic rat model, the glucagon analog 40 of the present disclosure induces higher blood glucose levels and demonstrates better efficacy at equivalent doses as compared with Dasiglucagon.

### Example 6. Efficacy assessment of glucagon analogs in hypoglycemic rat model

Hypoglycemia symptoms were induced by subcutaneous injection of insulin in rats to simulate a hypoglycemia scene in humans, and the glucagon analog was subcutaneously administered to quickly restore the normal blood glucose level.

### 1. Objective

Glucagon analog 40 of the present disclosure was assessed for efficacy in a hypoglycemic rat model in comparison to Dasiglucagon.

### 2. Procedures

Male SD rats (Sprague-Dawley rats) aged 7-8 weeks were acclimatized for one week. The rats were grouped according to the blood glucose level to ensure similar average blood glucose levels and standard deviations among groups. The animals were deprived of food 4 hours before and during the experiment, but were provided with free access to water. After 4 hours of fasting, the blood glucose level at T0 point was determined by tail blood sampling. The modeling groups were immediately administered with 0.65 IU/kg of insulin subcutaneously, and the non-modeling groups were administered with the same volume of normal saline subcutaneously. Then, the blood glucose was measured at 15 min, 30 min, and 45 min. At 45 min, the modeling group rats were administered with 6 nmol/kg, 12 nmol/kg or 20 nmol/kg of glucagon analog 40 or 12 nmol/kg of Dasiglucagon subcutaneously in a single dose, and the control group rats and non-modeling group rats were subcutaneously administered with the same phosphate buffer as for glucagon analog 40. Then, the blood glucose of the rats was continuously measured at 60 min, 75 min, 90 min, 105 min, 120 min, 150 min, and 180 min.

### 3. Results

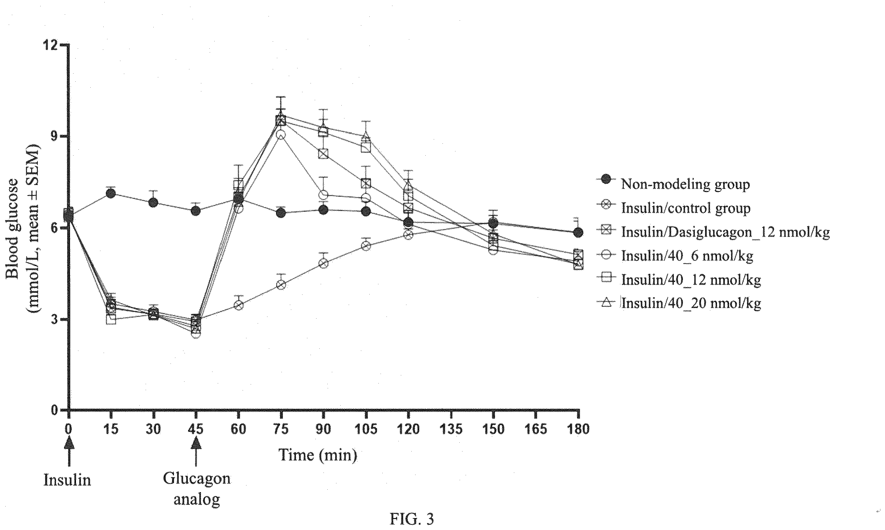

After insulin treatment, the administration of glucagon analog 40 of the present disclosure given at the nadir of rat blood glucose rapidly restored the normal blood glucose level in rats, showing dose-dependence. Although both glucagon analog 40 of the present disclosure and Dasiglucagon exhibited short-acting characteristics, glucagon analog 40 of the present disclosure demonstrated higher glycemic effects at the same dose. The specific results are shown in FIG. 3 and Table 8.

**Table 8. Hyperglycemic results of compounds in hypoglycemic rat models**

| Compounds (dose) | Blood glucose (mmol/L) | | | |
|---|---|---|---|---|
| | 60 min | 75 min | 90 min | 105 min |
| Insulin | 3.47 | 4.15 | 4.85 | 5.42 |
| Dasiglucagon (12 nmol/kg) | 7.00 | 9.52 | 8.43 | 7.47 |
| 40 (6 nmol/kg) | 6.65 | 9.05 | 7.08 | 6.99 |
| 40 (12 nmol/kg) | 7.40 | 9.50 | 9.13 | 8.63 |
| 40 (20 nmol/kg) | 6.87 | 9.68 | 9.28 | 9.00 |

### 4. Conclusion

The results show that glucagon analog 40, the native glucagon, and Dasiglucagon can rapidly restore the normal blood glucose levels in rats within 15 minutes after subcutaneous administration in an insulin-induced hypoglycemia rat model. At the same dose (12 nmol/kg), the effect of glucagon analog 40 on raising blood glucose level is significantly better than that of Dasiglucagon and lasts longer.

Glucagon analog 40 is more potent than Dasiglucagon in the insulin-induced hypoglycemia rat model at equivalent doses.

### Example 7. Hemolysis assessment of glucagon analogs

Hemolysis refers to the phenomenon in which the erythrocyte membrane is destroyed, along with increased transparency and a deep red color of blood. Some pharmaceutical materials, excipients, and the like may contain hemolytic components, which may cause hemolysis in a human body and cause adverse reactions such as local swelling, blood circulation dysfunction, and the like. According to the principle that the hemoglobin released from lysed erythrocytes has absorption in visible light wave band, test compound solutions were added into rat erythrocyte suspension, and after incubation, the absorbance was determined by a microplate reader to assess the hemolysis.

### 1. Objective

To observe whether glucagon analog 40 would cause hemolysis.

### 2. Procedures for hemolysis assay

Preparation of erythrocyte suspension: 100 µL of fresh rat whole blood was collected and added to 900 µL of 1 × PBS solution. The mixture was shaken on a plate shaker at 30 rpm for 5 min and centrifuged at 1000 g for 5 min before the supernatant was discarded. The above washing procedures were repeated until the supernatant was no longer in red color. The suspension was preserved for later use.

Preparation of test solutions: An appropriate amount of 1 × PBS solution was added to the test compound solid for dissolution, thus giving a standard stock solution. The stock solution was then diluted with 1 × PBS to give test solutions at concentrations of 1 µg/mL, 3 µg/mL, 10 µg/mL, 30 µg/mL, 100 µg/mL, and 300 µg/mL. Meanwhile, blank 1 × PBS was used as the negative control, and 1 × PBS solution containing 0.1% Triton X-100 (1 µg/mL) was used as the positive control.

Incubation: 500 µL of the test solution was added to the erythrocyte suspension. The mixture was shaken on a plate shaker at 30 rpm for 5 min to mix well, incubated at 37 °C for 1 hour, and centrifuged at 1000 g for 5 min. 100 µL of the supernatant was transferred to each well of a microplate, and the absorbance at 540 nm was determined.

### 3. Results

The hemolysis rate (%) was calculated according to the formula: (test sample absorbance - negative control absorbance)/(positive control absorbance - negative control absorbance) × 100%. A rate of less than 5% indicates the absence of hemolysis, while a rate of more than 5% indicates the presence.

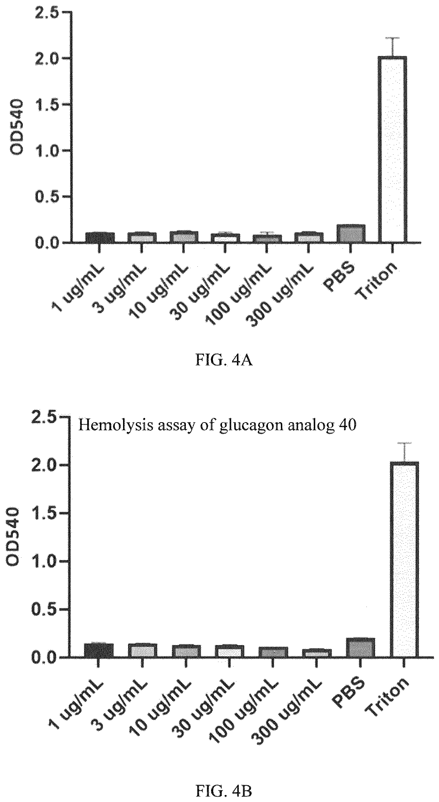

The hemolytic assay results for the glucagon analog of the present disclosure are shown in FIGs. 4A and 4B and Table 9. The results show that glucagon analog 40 of the present disclosure and Dasiglucagon, at concentrations ranging from 1 to 300 µg/mL, both met the regulatory requirements for hemolysis and no significant risk of the hemolysis adverse effect was found.

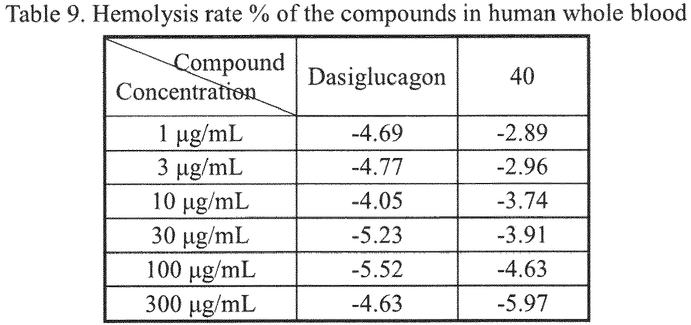

### Example 8. Stability assessment of glucagon analogs in human liver and kidney microsomes

The liver and kidneys are the major metabolic organs in humans, and are rich in phase I and phase II metabolic enzymes which may influence the metabolism of drugs. Thus the liver and kidneys play important roles in degrading and clearing drugs, and may influence the blood concentration and half-life of drugs. The common models for liver and kidney metabolism include liver and kidney microsomes, S9 fraction, liver cells, tissue homogenate, etc. The liver and kidney metabolic stability *in vitro* is one of the major indexes for predicting the pharmacokinetics and pharmacodynamics *in vivo.* The liver and kidney microsome models use microsomes extracted from the liver or kidneys and a reduced coenzyme (NADPH) regeneration system to simulate the metabolic reaction in a physiological environment, and to assess the *in vitro* metabolic stability of a test compound, and can be used for predicting the *in vivo* degradation and clearance speed of drugs. In proper conditions, the glucagon analog needs quick absorption and clearance and thus a relatively high degradation speed *in vivo*, so as to alleviate hypoglycemia and avoid hyperglycemic adverse effects.

### 1. Objective

To compare and assess the metabolic stability of glucagon analogs in the human liver and kidney microsomal *in vitro* metabolic system.

### 2. Procedures for human liver and kidney microsomal assessment

Preparation of solutions: An appropriate amount of purified compound was dissolved in an appropriate amount of DMSO to give 1 mM test solutions of Dasiglucagon and glucagon analog 40.

Preparation of MgCl₂-PBS buffer: 106 µL H₂O and 40 µL of MgCl₂ solution (50 mM) were sequentially added to 200 µL of 200 mM PBS buffer. The mixture was vortexed to mix well to give the MgCl₂-PBS buffer.

Procedures for stability assessment: The samples were processed according to the following procedures.
(1) Preparation of incubation system
   a) System with NADPH: 10 µL of 20 mg/mL liver microsome and 40 µL of 10 mM NADPH solution were added into the incubation system at final concentrations of 0.5 mg/mL and 1 mM, respectively;
   b) System without NADPH: 10 µL of 20 mg/mL liver microsome and 40 µL of H₂O were added to the incubation system.
(2) 4 µL of 100 µM test compound solution and positive control compound (verapamil for human liver microsome and benzydamine hydrochloride for human kidney microsome) at a final concentration of 1 µM and the system was incubated at 37 °C. Verapamil and benzydamine hydrochloride are known substrates of liver metabolic enzymes and kidney metabolic enzymes, respectively (see Pauli-Magnus C et al, J Pharmacol Exp Ther. 2000 May;293 (2): 376-82.) and are used for confirming the enzymatic activity of microsome incubation systems;
(3) Samples of 50 µL were taken at 0 min, 15 min, 30 min, 60 min, and 120 min, and 200 µL of acetonitrile solution containing internal standard and 3% formic acid was added to terminate the reaction. The sample was further centrifuged at 3220 g for 40 min. 100 µL of the supernatant was added with an equal volume of H₂O and the mixture was mixed well for HPLC-MS/MS analysis.

### 3. Results

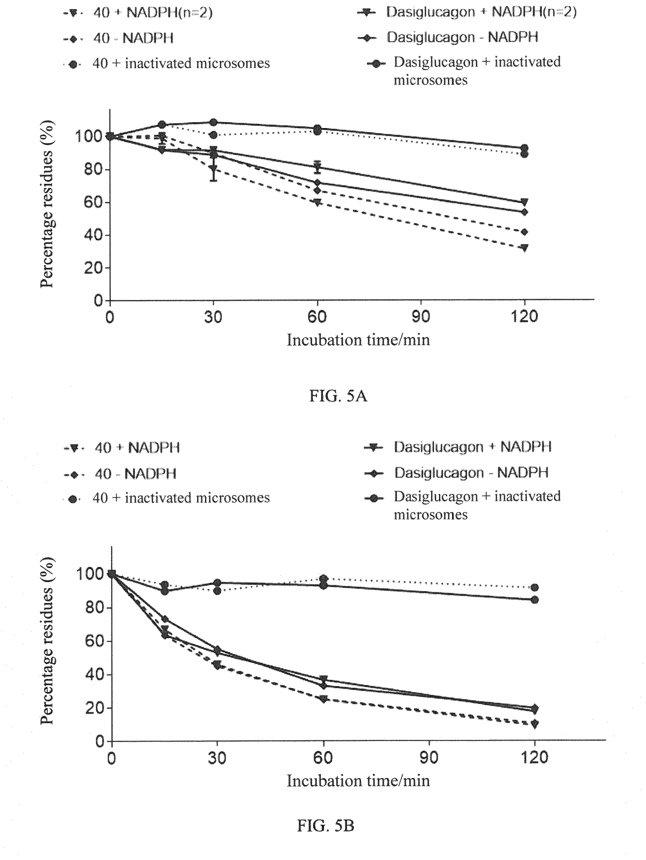

The stability assessment results for the glucagon analog disclosed herein in human liver microsomes and kidney microsomes are shown in FIGs. 5A and 5B and Tables 10 and 11.

**Table 10. Stability assessment results in human liver microsome incubation system**

| Compound | Detection system | T1/2 (min) | Percentage residues (%) | | | | |
|---|---|---|---|---|---|---|---|
| | | | 0 min | 15 min | 30 min | 60 min | 120 min |
| Verapamil | With NADPH | 9.97 | 100.0 | 16.27 | 5.34 | 1.31 | 0.16 |
| | Without NADPH | 1126 | 100.0 | 98.39 | 92.30 | 94.86 | 88.13 |
| Dasiglucagon | With NADPH | 161.74 | 100.0 | 91.84 | 91.58 | 81.12 | 59.22 |
| | Without NADPH | 125.37 | 100.0 | 91.65 | 88.78 | 71.74 | 53.48 |
| 40 | With NADPH | 68.76 | 100.0 | 98.74 | 80.14 | 59.43 | 31.52 |
| | Without NADPH | 125.37 | 100.0 | 100.5 | 89.85 | 66.96 | 41.48 |

**Table 11. Stability assessment results in human kidney microsome incubation system**

| Compound | Detection system | T1/2 (min) | Percentage residues (%) | | | | |
|---|---|---|---|---|---|---|---|
| | | | 0 min | 15 min | 30 min | 60 min | 120 min |
| Benzydamine hydrochloride | With NADPH | 66.2 | 100.0 | 83.10 | 70.79 | 54.24 | 28.12 |
| | Without NADPH | NA | 100.0 | 85.59 | 86.82 | 90.39 | 90.95 |
| Dasiglucagon | With NADPH | 51.27 | 100.0 | 63.55 | 52.86 | 36.65 | 17.73 |
| | Without NADPH | 51.98 | 100.0 | 73.24 | 55.17 | 33.12 | 19.74 |
| 40 | With NADPH | 35.73 | 100.0 | 66.85 | 45.98 | 24.96 | 9.38 |
| | Without NADPH | 38.19 | 100.0 | 63.34 | 45.04 | 25.16 | 10.61 |

### 4. Conclusion

In liver metabolism: firstly, Dasiglucagon and glucagon analog 40 are metabolized slower in liver microsomes than in kidney microsomes, and are metabolized independently of NADPH; secondly, glucagon analog 40 is degraded slightly faster than Dasiglucagon (the *in vitro* T_{1/2} was 68.76 min for glucagon analog 40, and 161.74 min for Dasiglucagon).

In kidney metabolism: first, Dasiglucagon and glucagon analog 40 are both metabolized in kidney microsomes independent of NADPH; secondly, glucagon analog 40 is degraded faster than Dasiglucagon (the *in vitro* T_{1/2} was 35.73 min for glucagon analog 40, and 51.27 min for Dasiglucagon).

In summary, glucagon analog 40 of the present disclosure is metabolized faster than Dasiglucagon in the liver and kidney microsome incubation. The compound is thus more readily cleared *in vivo*, possesses less risk of hyperglycemic adverse effects, and is more favorable for glycemic control in patients with diabetes.

### Example 9. Pharmacokinetic assessment of glucagon analogs in rats

The general method for animal pharmacokinetic study is to determine the variation of drug concentration in biological matrices such as blood, urine, and the like by HPLC, GC, HPLC-MS, and other analytic methods after the test compound is administered to an animal via the proposed route of administration for clinical use, thereby exploring the dynamic variation manner of the compound *in vivo,* plotting the blood concentration-time curve, acquiring the pharmacokinetic parameters of the drug, interpreting the absorption, distribution, metabolism, and excretion process and characteristics of the compound.

### 1. Objective

Dasiglucagon and glucagon analog 40 were administered by a single subcutaneous injection in male SD rats to investigate the pharmacokinetics of Dasiglucagon and glucagon analog 40 in SD rats (plasma).

### 2. Procedures

(1) Animals were grouped according to Table 12, and were given free access to water and food before administration.

**Table 12. Grouping**

| Group No. | Test compound | Dose (mg/kg) | Volume (mL/kg) | Concentration (mg/mL) | Route of administration | Animal |
|---|---|---|---|---|---|---|
| 1 | Dasiglucagon | 5 | 5 | 1.000 | Subcutaneous injection | 3 males |
| 2 | 40 | 5.06 | 5 | 1.012 | Subcutaneous injection | 3 males |

(2) Blood sampling
Sampling time: 0 min pre-dose, and 2 min, 4 min, 7 min, 10 min, 20 min, 40 min, 1 h and 2 h post-dose. Blood was collected from rats by jugular catheterization.
(3) Sample treatment
1) 0.2 mL of whole blood was collected at each time point into a centrifuge tube containing EDTA-K2;
2) The blood samples were centrifuged at 4000 g at 4 °C for 5 min, and separated to give the plasma;
3) The plasma samples were cryopreserved at -80 °C.

(4) Biological sample analysis

Sample pretreatment: 100 µL of plasma sample was added to 300 µL of 1 ng/mL internal standard (verapamil) in methanol. The mixture was vortexed for 5 min and centrifuged at a low temperature for 10 min. 100 µL of the supernatant was taken and added to 100 µL of 0.1% formic acid aqueous solution. The mixture was mixed well, and loaded on the instrument for analysis;
Liquid chromatography and mass spectrometry analysis: LC-30A liquid chromatography system (Shimadzu, Japan) and API 5500 liquid chromatography-triple quadrupole mass spectrometer (Analyst Software data processing system, AB Sciex, USA) were used. The chromatographic column was nanomicro Unisil C18aq (4.6 mm × 150 mm, 5 µm) chromatographic column; the column temperature was 40 °C; the mobile phase A was 0.1% formic acid aqueous solution, and the mobile phase B was 0.1% formic acid in acetonitrile; the flow rate was 0.5 mL/min; the injection volume was 20 µL; the gradient is shown in Table 13 below. The mass spectrum was obtained using an electrospray ionization (ESI) source, a positive ion analysis mode and a multi reaction monitoring (MRM) scanning mode.

**Table 13. LC gradient program**

| Time (min) | Phase A (%) | Phase B (%) |
|---|---|---|
| 0.5 | 95 | 5 |
| 5.5 | 5 | 95 |
| 8.5 | 5 | 95 |
| 8.51 | 95 | 5 |
| 10.0 | 95 | 5 |

### 3. Results

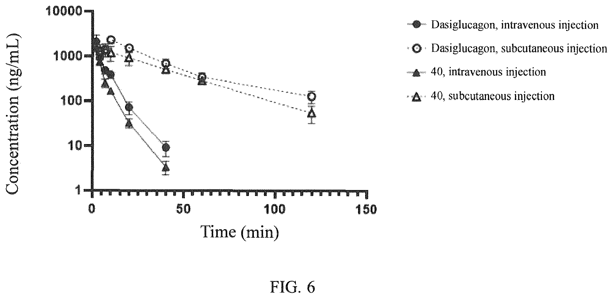

The plasma concentration of the test compounds was measured by HPLC-MS/MS. The plasma concentration-time curve was plotted, and the pharmacokinetic parameters were calculated by PKSolver. By the above procedures, pharmacokinetic (PK) parameters of the glucagon analog disclosed herein are shown in Table 14 and FIG. 6.

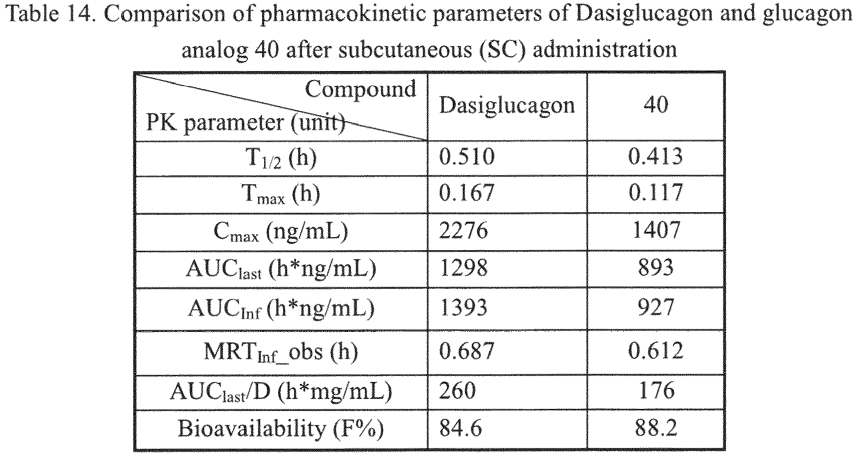

### 4. Conclusion

The results show that, after subcutaneous administration, the T_{1/2} of glucagon analog 40 was significantly shorter than that of Dasiglucagon (the T_{1/2} was 0.41 h for glucagon analog 40 and 0.51 h for Dasiglucagon), indicating a faster clearance rate for glucagon analog 40; the Tₘₐₓ for glucagon analog 40 was also shorter than that of Dasigucagon (the Tₘₐₓ was 0.117 h for glucagon analog 40 and 0.167 h for Dasiglucagon), indicating that the absorption rate of glucagon analog 40 is significantly higher than that of Dasigucagon.

In summary, glucagon analog 40 can be absorbed faster and cleared faster than Dasiglucagon, with superior pharmacokinetics.

## Claims

1. A glucagon analog, or a pharmaceutically acceptable salt and/or solvate thereof, having a structure of formula (I): 
R1-His-Ser-X3-Gly-Thr-Phe-Thr-Ser-Asp-Tyr-Ser-Lys-Tyr-Leu-X15-X16-X17-Arg-Ala-
X20-X21-Phe-Val-X24-Trp-Leu-X27-X28-Thr-R2
(I)
 wherein:
R₁ is hydrogen, C₁₋₄ alkyl, acetyl, formyl, benzoyl, trifluoroacetyl, or pGlu;
R₂ is -OH or -NH₂;
X₃, X₁₅, X₁₆, X₂₀, X₂₁, X₂₄, X₂₇, and X₂₈ are independently selected from the group consisting of any natural and unnatural amino acid residues; X₁₇ is Aib.

2. The glucagon analog, or the pharmaceutically acceptable salt and/or solvate thereof according to claim 1, wherein:
X₃ is selected from the group consisting of His, Dap (Ac), and Gln;
X₁₅ is selected from the group consisting of Asp and Glu;
X₁₆ is selected from the group consisting of Ser, Thr, Leu, Val, Ile, and alpha-methyl-Ser;
X₂₀ is selected from the group consisting of Ala, Gln, Glu, Ser, Thr, and Lys;
X₂₁ is selected from the group consisting of Asp and Glu;
X₂₄ is selected from the group consisting of Ala, Gin, Ser, Glu, alpha-methyl-Ser, and Arg;
X₂₇ is selected from the group consisting of Met, Glu, Nle, Leu, and Ser;
X₂₈ is selected from the group consisting of Asn, Glu, and Ser.

3. The glucagon analog, or the pharmaceutically acceptable salt and/or solvate thereof according to claim 1 or 2, wherein:
X₁₅ is Asp;
X₁₆ is selected from the group consisting of Thr, Leu, Val, and Ile.

4. The glucagon analog, or the pharmaceutically acceptable salt and/or solvate thereof according to any of claims 1-3, wherein: X₂₀ is Gln.

5. The glucagon analog, or the pharmaceutically acceptable salt and/or solvate thereof according to any of claims 1-4, wherein: X₂₄ is selected from the group consisting of Gln and Glu.

6. The glucagon analog, or the pharmaceutically acceptable salt and/or solvate thereof according to any of claims 1-4, wherein: X₂₇ is Glu; X₂₈ is Ser.

7. The glucagon analog, or the pharmaceutically acceptable salt and/or solvate thereof according to any of claims 1-6, wherein:
X₃ is selected from the group consisting of His and Gln;
X₁₅ is Asp;
X₁₆ is selected from the group consisting of Thr, Leu, Val, and Ile;
X₁₇ is Aib;
X₂₀ is Gin;
X₂₁ is Glu;
X₂₄ is selected from the group consisting of Gln and Glu;
X₂₇ is Glu;
X₂₈ is Ser.

8. The glucagon analog, or the pharmaceutically acceptable salt and/or solvate thereof according to claim 7, wherein:
R₁ is hydrogen; R₂ is -OH or -NH₂, preferably -OH.

9. The glucagon analog, or the pharmaceutically acceptable salt and/or solvate thereof according to any of claims 1-8, wherein the glucagon analog is selected from the group consisting of any of the following compounds:
H-HSQGTFTSDYSKYLDLAibRAQEFVQWLEST-OH (SEQ ID NO: 40);
H-HSQGTFTSDYSKYLDTAibRAQEFVQWLEST-OH (SEQ ID NO: 39);
H-HSQGTFTSDYSKYLDVAibRAQEFVQWLEST-OH (SEQ ID NO: 41);
H-HSQGTFTSDYSKYLDIAibRAQEFVQWLEST-OH (SEQ ID NO: 42);
H-HSHGTFTSDYSKYLDLAibRAQEFVQWLEST-OH (SEQ ID NO: 43); and
H-HSHGTFTSDYSKYLDLAibRAQEFVEWLEST-OH (SEQ ID NO: 47).

10. A pharmaceutical composition comprising:
the glucagon analog, or the pharmaceutically acceptable salt and/or solvate thereof according to any of claims 1-9, and
one or more pharmaceutically acceptable carriers, diluents, buffers, or excipients.

11. The pharmaceutical composition according to claim 10, further comprising at least one compound having therapeutic activity for a metabolic disease.

12. The pharmaceutical composition according to claim 11, wherein the compound having therapeutic activity for a metabolic disease is selected from the group consisting of one or more of the following: glucose-dependent insulinotropic polypeptide (GIP), a glucagon-like peptide-1 (GLP-1) receptor agonist, insulin, enteroglucagon, a neuropeptide Y5 receptor antagonist, an acetyl-CoA carboxylase inhibitor, a leptin receptor agonist, a glinide, an alpha-glucosidase inhibitor (AGi), a thiazolidinedione (TZD), a dipeptidyl peptidase-4 inhibitor (DPP-IV), a sodium-glucose cotransporter 2 (SGLT-2) inhibitor, a farnesol X receptor (FXR) agonist, and obestatin.

13. Use of the glucagon analog, or the pharmaceutically acceptable salt and/or solvate thereof according to any of claims 1-9, or the pharmaceutical composition according to any of claims 10-12 in preparing a medicament for treating a disease or condition; preferably, the disease or condition is selected from the group consisting of hypoglycemia, hyperglycemia, type 2 diabetes mellitus, type 1 diabetes mellitus, coronary heart disease, atherosclerosis, beta-blocker toxicity, insulinoma, Von Gierke disease, impaired glucose tolerance, dyslipidemia, hypertension, overweight, binge eating, and hepatic steatosis.

14. The use according to claim 13, wherein the hypoglycemia is pathological hypoglycemia or non-pathological hypoglycemia.

15. The use according to claim 13, wherein the hypoglycemia is selected from the group consisting of diabetic hypoglycemia, non-diabetic hypoglycemia, fasting hypoglycemia, drug-induced hypoglycemia, acute insulin-induced hypoglycemia, gastric bypass-induced hypoglycemia, alcohol-induced hypoglycemia, reactive hypoglycemia, and gestational hypoglycemia.

16. A method for preparing the glucagon analog, or the pharmaceutically acceptable salt and/or solvate thereof according to any of claims 1-9, comprising: preparing the glucagon analog, or the pharmaceutically acceptable salt and/or solvate thereof by solid-phase synthesis, liquid-phase synthesis, or recombinant expression in cells.
